# EUROPEAN PATENT APPLICATION

(11) **EP 4 397 694 A1**
(43) Date of publication of application: **10.07.2024**
(21) Application number: 22864594.1
(22) Date of filing: 31.08.2022
(51) Int. Cl.: C08G 77/30, A61F 2/16, A61L 27/16, C08F 290/06, C08G 77/395, G02C 7/00, G02C 7/04

(54) **POLYDIMETHYLSILOXANE-CONTAINING MONOMER HAVING PHOSPHORYLCHOLINE GROUP AND HYDROXYL GROUP**

(30) Priority: 31.08.2021 JP 2021141694
(71) Applicant: NOF Corporation, Shibuya-ku Tokyo 150-6019 (JP)
(72) Inventor: TANAKA Yoshiki, Kawasaki-shi, Kanagawa 210-0865 (JP); TAKASHIMA Shu, Kawasaki-shi, Kanagawa 210-0865 (JP); IWAKIRI Norio, Kawasaki-shi, Kanagawa 210-0865 (JP)
(74) Representative: Mewburn Ellis LLP
(86) International application number: PCT/JP2022/032689
(87) International publication number: WO 2023/033012

(57) **Abstract**

An object is to provide a polydimethylsiloxane-containing monomer that exhibits satisfactory compatibility with a hydrophilic monomer and a hydrophilic polymer, in which a polymerization product obtained by polymerizing a composition including the polydimethylsiloxane-containing monomer, the hydrophilic monomer, and the hydrophilic polymer has an anti-lipid adhesion property. It has been recognized that a polydimethylsiloxane-containing monomer having a phosphorylcholine group and a hydroxy group can achieve the object, and thus the present disclosure has been completed.

## Description

### Technical Field

The present disclosure relates to a polydimethylsiloxane-containing monomer having a phosphorylcholine group and a hydroxy group, a method of producing the monomer, a composition including the monomer, a polymerization product obtained by polymerizing the composition, an ophthalmic device including the polymerization product, and a method of producing a polydimethylsiloxane-containing monomer having a hydrosilane at a terminal thereof.

The present application claims priority from Japanese Patent Application No. 2021-141694, which is incorporated herein by reference.

### Background Art

In order to maintain the health state of the cornea, a contact lens that is an ophthalmic device needs to receive the supply of oxygen from air and requires oxygen permeability. In recent years, a contact lens using a siloxane monomer as a raw material has been developed in order to improve the oxygen permeability.

A contact lens requires wettability in addition to the oxygen permeability. It is said that a contact lens having high wettability has satisfactory wearing sensation and can be worn comfortably for a long period of time. In order to improve the wettability of a contact lens, a hydrophilic monomer is generally incorporated into a raw material.

In order to produce a contact lens having both high oxygen permeability and high wettability, there has been used a method including using both the siloxane monomer and the hydrophilic monomer as raw materials. However, the siloxane monomer generally has high hydrophobicity and has poor compatibility with the hydrophilic monomer. Thus, phase separation is liable to occur, resulting in difficulty in producing a transparent contact lens. In addition, an excellent contact lens needs to satisfy a plurality of elements, such as suitable mechanical strength, in addition to high oxygen permeability and high wettability. In order to satisfy those elements simultaneously, various investigations have been made on kinds and blending ratios of the siloxane monomer and the hydrophilic monomer with a view to optimizing formulation.

However, also in the investigations on formulation, compatibility between the siloxane monomer and the hydrophilic monomer and the accompanying transparency of a contact lens are problems, and hence there is a demand for a siloxane monomer having such high compatibility as to allow the siloxane monomer to be mixed with various hydrophilic monomers.

In order to produce a contact lens having both high oxygen permeability and high wettability, there has been known a method including mixing a contact lens composition with a hydrophilic polymer.

In Patent Literature 1, a contact lens produced through use of a composition mixed with a hydrophilic polymer such as polyvinylpyrrolidone (PVP) in addition to the siloxane monomer and the hydrophilic monomer exhibits more satisfactory wearing sensation because of high wettability. Meanwhile, there is a problem in that the hydrophilic polymer also has poor compatibility with the siloxane monomer similarly to the hydrophilic monomer, and hence phase separation is liable to occur.

In order to solve those problems, a siloxane monomer subjected to hydrophilization has been developed. For example, in Patent Literatures 2 and 3, there is a disclosure of a contact lens produced from a polydimethylsiloxane-containing monomer having a hydrophilic group. A polyether group, a hydroxy group, an amide group, or the like is selected as the hydrophilic group. However, in order to achieve high compatibility with the hydrophilic monomer and the hydrophilic polymer, it is required to introduce a large amount of the hydrophilic group with respect to polydimethylsiloxane, and hence there are concerns about adverse influences on other elements required for an excellent contact lens.

### Citation List

### Patent Literature

[PTL 1] US 6822016 B2
[PTL 2] JP 62-29776 B2
[PTL 3] JP 62-29777 B2

### Summary of Invention

### Technical Problem

A contact lens using a siloxane monomer as a raw material exhibits high oxygen permeability. Meanwhile, one of the problems of such contact lens is that lipid adhesion is liable to occur due to the hydrophobicity of the siloxane monomer, and the lipid adhesion causes a decrease in wearing sensation. A method such as surface modification is used to suppress the lipid adhesion. For example, a production process for a contact lens includes plasma treatment in some cases. However, the plasma treatment requires a dedicated facility, and hence there is a demand for a simpler surface modification method from the viewpoint of cost.

In view of the foregoing, an object of the present disclosure is to provide a polydimethylsiloxane-containing monomer that exhibits satisfactory compatibility with a hydrophilic monomer and a hydrophilic polymer, in which a contact lens obtained by hydrating a polymerization product, which is obtained by polymerizing a composition including the polydimethylsiloxane-containing monomer and the hydrophilic monomer, has excellent surface hydrophilicity and coatability, and in which a contact lens obtained by hydrating a polymerization product, which is obtained by polymerizing a composition including the polydimethylsiloxane-containing monomer, the hydrophilic monomer, and the hydrophilic polymer, has an anti-lipid adhesion property.

### Solution to Problem

In the present disclosure, it has been recognized that a polydimethylsiloxane-containing monomer having a phosphorylcholine group and a hydroxy group represented by the following formula (1A) can achieve the object, and thus the present disclosure has been completed.

That is, the present disclosure is as described below.
1. A polydimethylsiloxane-containing monomer having a phosphorylcholine group and a hydroxy group represented by the formula (1A) (compound represented by the formula (1A)): 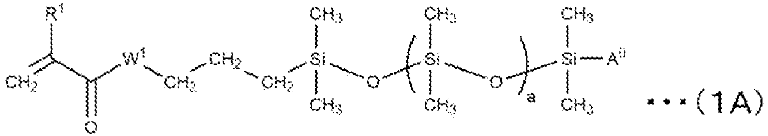 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 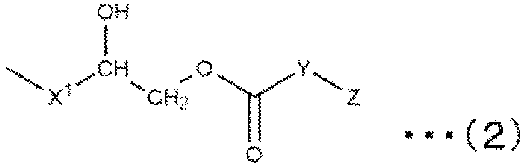 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 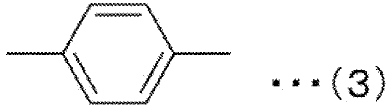 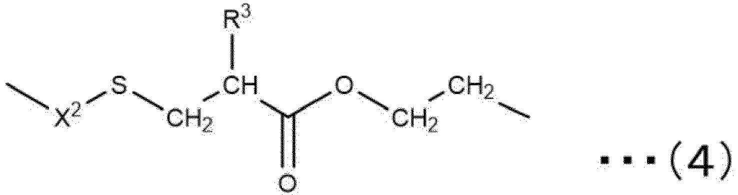 where R³ represents H or CH₃, X² represents a divalent alkylene group having 1 to 10 carbon atoms or a divalent group of -R¹⁰-O-R¹¹-, where R¹⁰ and R¹¹ each independently represent a divalent alkylene group having 1 to 6 carbon atoms.
2. A method of producing a compound represented by the formula (1A), the method including a step of synthesizing the formula (1A) by: a hydrosilylation reaction between a compound represented by the formula (6A) and a vinyl compound having an epoxy group, and further a reaction between an epoxy group of a compound after the reaction and a carboxylic acid of a carboxylic acid compound having a phosphorylcholine group; or a reaction between an epoxy group of a vinyl compound having an epoxy group and a carboxylic acid of a carboxylic acid compound having a phosphorylcholine group, and further a hydrosilylation reaction between a vinyl compound having a phosphorylcholine group and a hydroxy group after the reaction and the compound represented by the formula (6A): 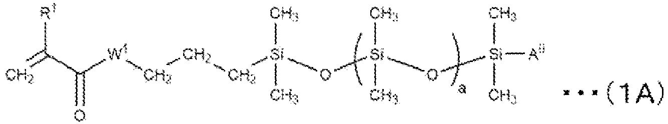 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 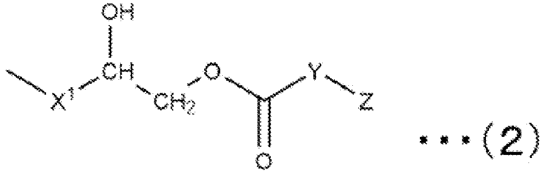 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 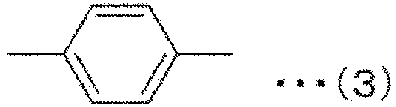 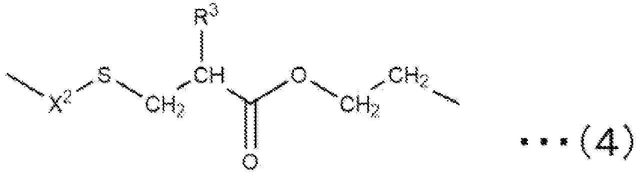 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms; 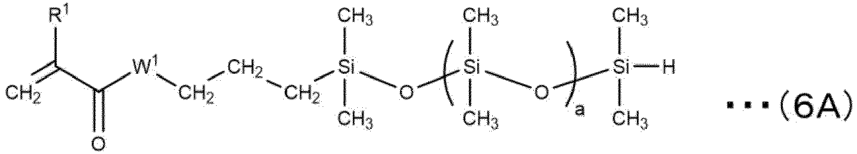 where R¹ represents H or CH₃, W¹ represents O or NR⁴, where R⁴ represents H or an alkyl group having 1 to 4 carbon atoms, and "a" represents an integer of from 1 to 100.
3. A method of producing a polydimethylsiloxane-containing monomer having a hydrosilane at a terminal thereof represented by the formula (6A), the method including a step of synthesizing the polydimethylsiloxane-containing monomer having a hydrosilane at a terminal thereof represented by the formula (6A) by: converting a chlorosilane compound represented by the formula (7) into a silanol compound represented by the formula (7A) with a metal hydroxide; and then allowing the silanol compound to react with a cyclic siloxane and a chlorosilane compound in the presence of an organic base: 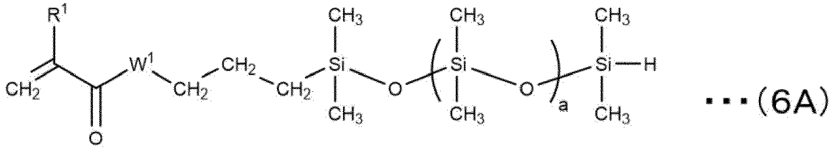 where R¹ represents H or CH₃, W¹ represents O or NR⁴, where R⁴ represents H or an alkyl group having 1 to 4 carbon atoms, and "a" represents an integer of from 1 to 100; 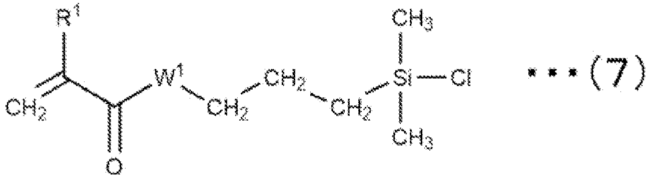 where R¹ represents H or CH₃, W¹ represents O or NR⁴, where R⁴ represents H or an alkyl group having 1 to 4 carbon atoms; 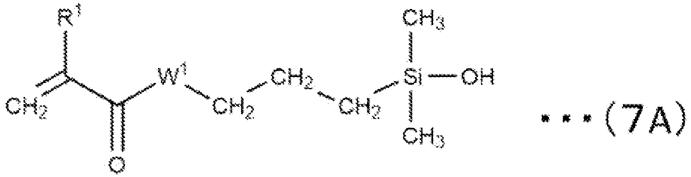 where R¹ represents H or CH₃, W¹ represents O or NR⁴, where R⁴ represents H or an alkyl group having 1 to 4 carbon atoms.
4. A composition, including: a compound represented by the following formula (1A); and one or more kinds of hydrophilic monomers: 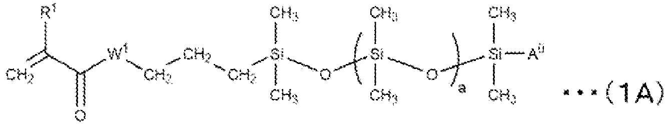 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 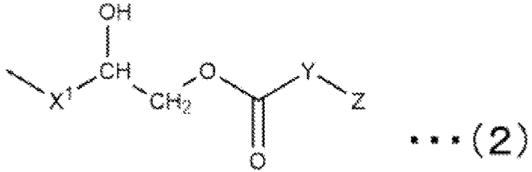 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 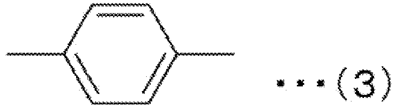 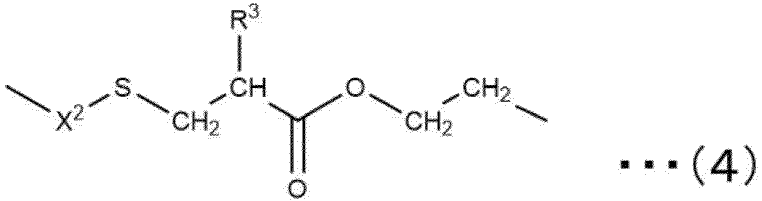 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.
5. A composition, including: a compound represented by the following formula (1A); one or more kinds of hydrophilic monomers; and one or more kinds of hydrophilic polymers: 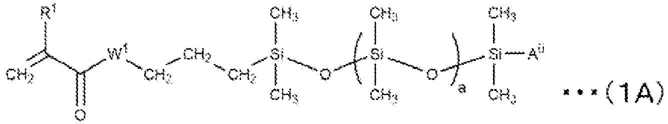 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 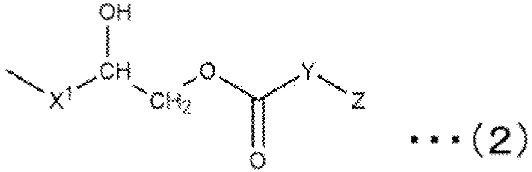 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 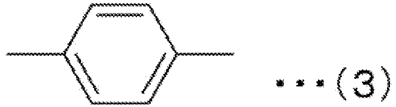 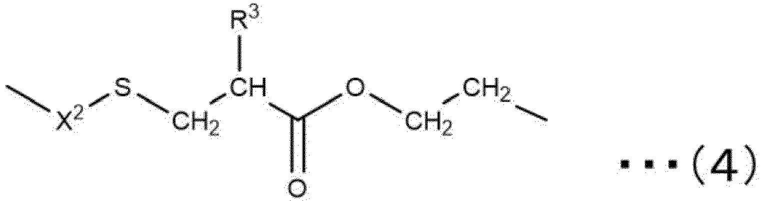 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.
6. A composition, including: a compound represented by the following formula (1A); one or more kinds of hydrophilic monomers; and one or more kinds of siloxane monomers: 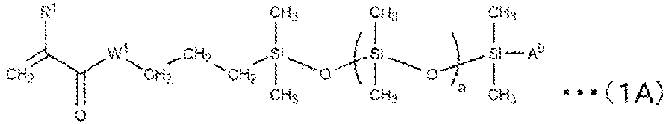 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 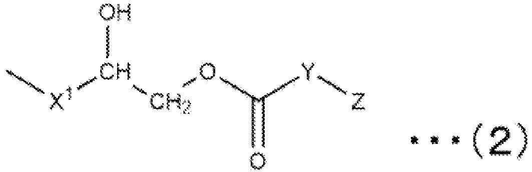 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 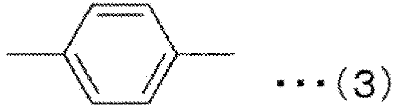 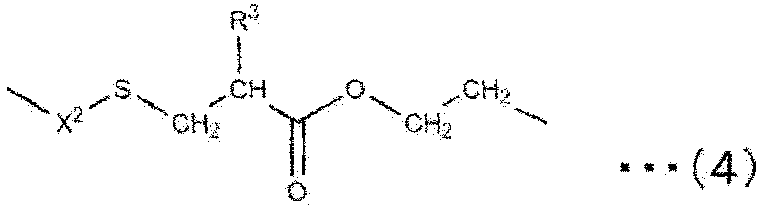 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.
7. The composition according to any one of the above-mentioned items 4 to 6, wherein the hydrophilic monomer is 2-(methacryloyloxyethyl)-2-(trimethylammonioethyl) phosphate.
8. A composition, including: a compound represented by the following formula (1A); one or more kinds of hydrophilic monomers; one or more kinds of hydroxy group-containing siloxane monomers; and one or more kinds of hydrophilic polymers: 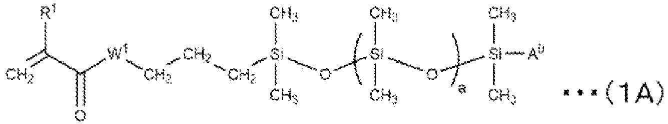 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 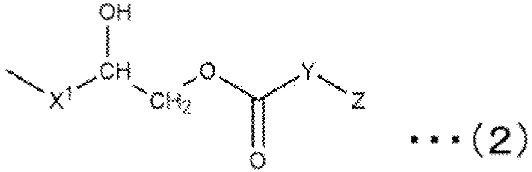 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 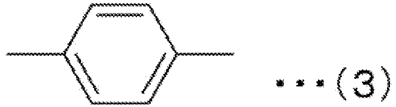 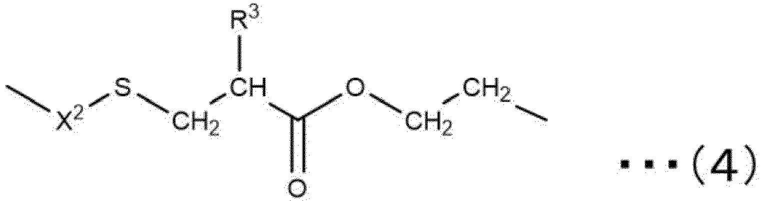 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.
9. The composition according to any one of the above-mentioned item 5 or 8, wherein the hydrophilic polymer is one or more selected from the group consisting of: polyamide; polylactam; polyimide; polylactone; and polydextran.
10. The composition according to any one of the above-mentioned item 5 or 8, wherein the hydrophilic polymer is polyvinylpyrrolidone.
11. A polymerization product obtained by polymerizing the composition of any one of the above-mentioned items 4 to 6 and 8.
12. An ophthalmic device, including the polymerization product of the above-mentioned item 11.
13. The compound represented by the formula (1A) according to the above-mentioned item 1, wherein the R¹ represents CH₃, the W¹ represents O, X¹ of the A⁰ represents CH₂CH₂CH₂-O-CH₂ or CH₂CH₂CH₂CH₂, and Y of the A⁰ is represented by the formula (3) or the formula (4) (where R³ represents CH₃ and X² represents CH₃) or represents CH₂.
14. The composition according to any one of the above-mentioned items 4 to 8, wherein the R¹ represents CH₃, the W¹ represents O, X¹ of the A⁰ represents CH₂CH₂CH₂-O-CH₂ or CH₂CH₂CH₂CH₂, and Y of the A⁰ is represented by the formula (3) or the formula (4) (where R³ represents CH₃ and X² represents CH₃) or represents CH₂.
15. A use of the composition of any one of the above-mentioned items 4 to 8 for producing an ophthalmic device.

### Advantageous Effects of Invention

The polydimethylsiloxane-containing monomer having a phosphorylcholine group and a hydroxy group of the present disclosure has satisfactory compatibility with a hydrophilic monomer and a hydrophilic polymer because of the presence of the phosphorylcholine group and the hydroxy group. A contact lens obtained by hydrating a polymerization product, which is obtained by polymerizing a composition including the polydimethylsiloxane-containing monomer and the hydrophilic monomer, has excellent surface hydrophilicity and coatability, and a contact lens obtained by hydrating a polymerization product, which is obtained by polymerizing a composition including the polydimethylsiloxane-containing monomer, the hydrophilic monomer, and the hydrophilic polymer, has transparency and an anti-lipid adhesion property.

### Brief Description of Drawings

FIG. 1 shows attribution results of ¹H NMR analysis of Example 1-1.
FIG. 2 shows attribution results of ¹H NMR analysis of Example 1-2.

### Description of Embodiments

The present disclosure relates to a polydimethylsiloxane-containing monomer having a phosphorylcholine group and a hydroxy group. More specifically, the polydimethylsiloxane-containing monomer having a phosphorylcholine group and a hydroxy group of the present disclosure relates to a polydimethylsiloxane compound which is represented by the following formula (1A), contains a polydimethylsiloxane moiety, and further contains a phosphorylcholine group and a hydroxy group in a molecule thereof, and further a vinyl terminal group, and which is preferably polymerizable.

The polydimethylsiloxane-containing monomer having a phosphorylcholine group and a hydroxy group of the present disclosure exhibits satisfactory compatibility with a hydrophilic monomer and a hydrophilic polymer because of the presence of the phosphorylcholine group and the hydroxy group in a molecule thereof.

When a composition of the polydimethylsiloxane-containing monomer and the hydrophilic monomer is polymerized, a transparent polymerization product is obtained. Further, a contact lens obtained by hydrating the polymerization product exhibits excellent surface hydrophilicity and coatability.

When a composition of the polydimethylsiloxane-containing monomer, the hydrophilic monomer, and the hydrophilic polymer is polymerized, a transparent polymerization product is obtained. Further, a contact lens obtained by hydrating the polymerization product exhibits an excellent anti-lipid adhesion property. In addition, when the polydimethylsiloxane-containing monomer has a vinyl terminal group, polymerization with any other composition (including monomers) becomes easy.

### <Polydimethylsiloxane-containing Monomer having Phosphorylcholine Group and Hydroxy Group of the Present Disclosure>

The polydimethylsiloxane-containing monomer having a phosphorylcholine group and a hydroxy group of the present disclosure is a polydimethylsiloxane-containing monomer represented by the formula (1A).

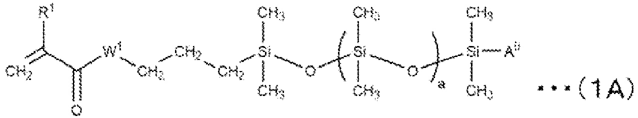

In the formula, R¹ represents H or CH₃, W¹ represents O or NR⁴, where R⁴ represents H or an alkyl group having 1 to 4 carbon atoms (e.g., a methyl group, an ethyl group, or a propyl group), "a" represents an integer of from 1 to 100, and A⁰ is represented by the formula (2).

"a" is not particularly limited as long as "a" falls within the above-mentioned range, but "a" represents from 1 to 100, preferably from 2 to 50, more preferably from 2 to 30, still more preferably from 2 to 15, particularly preferably from 2 to 10.

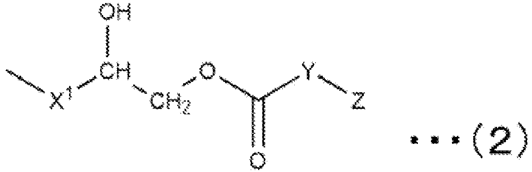

In the formula, X¹ represents a divalent alkylene group having 3 to 8 carbon atoms or a divalent group of -R⁶-OR⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group.

As used herein, the term "alkylene having 2 to 6 carbon atoms" refers to a divalent group obtained by removing two hydrogen atoms from an alkyl having 2 to 6 carbon atoms. The same also applies to other similar terms. An alkylene group forms two bonds with another group in an organic compound.

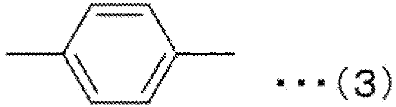

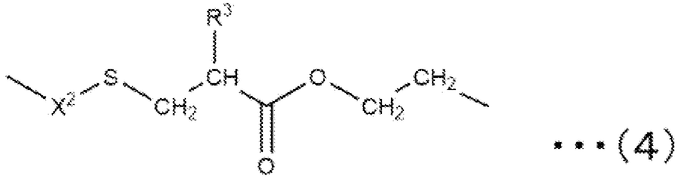

In the formula, R³ represents H or CH₃, X² represents a divalent alkylene group having 1 to 10 carbon atoms or a divalent group of -R¹⁰-O-R¹¹-, where R¹⁰ and R¹¹ each independently represent a divalent alkylene group having 1 to 6 carbon atoms.

Z represents a phosphorylcholine group, and may be exemplified by the following formula (5).

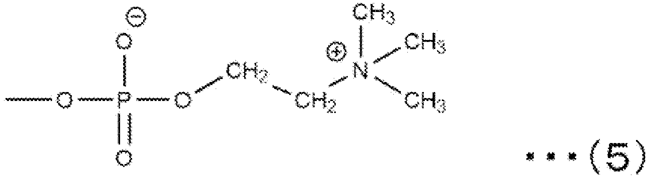

### <Method of producing Polydimethylsiloxane-containing Monomer having Phosphorylcholine Group and Hydroxy Group of the Present Disclosure>

The polydimethylsiloxane-containing monomer having a phosphorylcholine group and a hydroxy group represented by the formula (1A) of the present disclosure is synthesized by the following method.

The polydimethylsiloxane-containing monomer having a phosphorylcholine group and a hydroxy group represented by the formula (1A) is produced through: a step of synthesizing a polydimethylsiloxane-containing monomer having a hydrosilane at a terminal thereof represented by the formula (6A) by activating a chlorosilane compound represented by the formula (7) with a metal hydroxide to convert the chlorosilane compound into a silanol compound represented by the formula (7A) and allowing the silanol compound to react with a cyclic siloxane and a chlorosilane compound in the presence of an organic base; and a step of synthesizing the formula (1A) from the compound represented by the formula (6A) by combining a hydrosilylation reaction and a reaction between an epoxy group and a carboxylic acid. More specifically, the polydimethylsiloxane-containing monomer is produced through a step of synthesizing the formula (1A) by: a hydrosilylation reaction between the compound represented by the formula (6A) and a vinyl compound having an epoxy group, and further a reaction between an epoxy group of a compound after the reaction and a carboxylic acid of a carboxylic acid compound having a phosphorylcholine group; or a reaction between an epoxy group of a vinyl compound having an epoxy group and a carboxylic acid of a carboxylic acid compound having a phosphorylcholine group, and further a hydrosilylation reaction between a vinyl compound having a phosphorylcholine group and a hydroxy group after the reaction and the compound represented by the formula (6A).

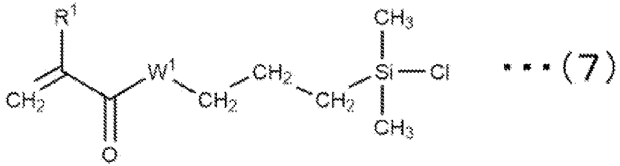

In the formula, R¹ represents H or CH₃, and W¹ represents O or NR⁴, where R⁴ represents H or an alkyl group having 1 to 4 carbon atoms.

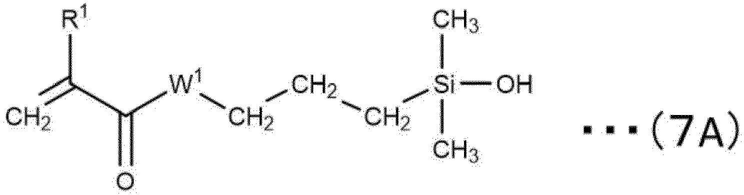

In the formula, R¹ represents H or CH₃, and W¹ represents O or NR⁴, where R⁴ represents H or an alkyl group having 1 to 4 carbon atoms.

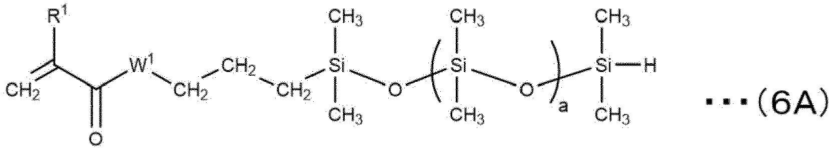

In the formula, R¹ represents H or CH₃, W¹ represents O or NR⁴, where R⁴ represents H or an alkyl group having 1 to 4 carbon atoms, and "a" represents an integer of from 1 to 100.

### <Method of producing Compound represented by Formula (6A)>

The compound represented by the formula (6A) is synthesized by the following method.

The chlorosilane compound represented by the formula (7) is dissolved in a solvent, and a metal hydroxide is added to convert the chlorosilane compound into the silanol compound represented by the formula (7A). After that, a poor solvent is added, and the precipitated metal salt is removed by filtration.

Subsequently, the cyclic siloxane is mixed with the resultant, and an organic base catalyst is added to initiate a reaction. Then, the reaction is terminated with a chlorosilane compound. Thus, the compound represented by the formula (6A) is synthesized. In this case, "a" in the formula (6A) can be obtained at a desired value by controlling a molar ratio between the silanol compound and the cyclic siloxane to be mixed.

Examples of the metal hydroxide include lithium hydroxide, sodium hydroxide, potassium hydroxide, magnesium hydroxide, and potassium hydroxide. Of those, lithium hydroxide is preferred from the viewpoint that a side reaction is less liable to occur and a metal salt is easily removed.

The metal hydroxide is added in an amount of typically from 1.0 mol equivalent to 4.0 mol equivalents, preferably from 1.5 mol equivalents to 2.5 mol equivalents with respect to the chlorosilane compound.

Examples of the solvent include dichloromethane, tetrahydrofuran, dimethylsulfoxide, dimethylformamide, and mixed solvents thereof. Of those, tetrahydrofuran is preferred from the viewpoint of solubility.

The solvent is used in an amount of typically from 5 equivalents to 50 equivalents, preferably from 10 equivalents to 30 equivalents with respect to the metal hydroxide.

Examples of the poor solvent include hexane, heptane, cyclohexane, 2-methylpentane, 3-methylpentane, benzene, toluene, and mixed poor solvents thereof. Of those, hexane and heptane are particularly preferred.

The poor solvent is used in an amount of typically from 0.5 equivalent to 1.5 equivalents, preferably from 0.8 equivalent to 1.2 equivalents with respect to the solvent.

6-Membered, 8-membered, and 10-membered cyclic siloxanes are each used as the cyclic siloxane. Of those, a 6-membered cyclic siloxane (hexamethylcyclotrisiloxane) is preferred from the viewpoint of reactivity.

In addition, when the cyclic siloxane is mixed, a solvent may be added. An example of the solvent to be added in this case is the above-mentioned solvent, and the same solvent is preferably used. The amount of the solvent to be added is not particularly limited, but is preferably from 1.0 equivalent to 10 equivalents, preferably from 1.5 equivalents to 5 equivalents with respect to the cyclic siloxane.

The organic base catalyst is an organic amine having, for example, an amidine structure, a guanidine structure, a phosphazene structure, or a proazaphosphatrane structure, and examples thereof include 1,5-diazabicyclo[4.3.0]non-5-ene (DBN), 1,8-diazabicyclo[5.4.0]undec-7-ene (diazabicycloundecene, DBU), 7-methyl-1,5,7-triazabicyclo[4.4.0]dec-5-ene (MTBD), 1,5,7-triazabicyclo[4.4.0]dec-5-ene (TBD), t-butylimino-tris(dimethylamino)phosphorane (BEMP), phosphazene base P1-tBu-tris(tetramethylene) (tBu-P1(Pyrr)), phosphazene base P2-Et (P2-Et)), and 2,8,9-triisobutyl-2,5,8,9-tetraaza-1-phosphabicyclo [3.3.3] undecane (TiBP). Of those, DBN and DBU are preferred.

The organic base catalyst exhibits its effect when added in a catalytic amount, and hence is typically added in an amount of from 0.01 mol% to 0.5 mol%, preferably from 0.05 mol% to 0.2 mol% with respect to the silanol group-containing compound represented by the formula (7A).

The organic base catalyst may be removed by liquid separation purification after the reaction.

A chlorosilane compound having a hydrosilane is used as the chlorosilane compound, and examples thereof include chlorodimethylsilane, 1-chloro-1,1,3,3-tetramethyldisiloxane, 1-chloro-1,1,3,3,5,5-hexamethyltrisiloxane, and 1-chloro-1,1,3,3,5,5,7,7-octamethyltetrasiloxane. Of those, chlorodimethylsilane is preferred from the viewpoint of availability.

When the reaction is terminated with the chlorosilane compound, it is required that the chlorosilane compound be added in a molar amount equal to or more than that of the silanol compound represented by the formula (7A). Thus, the chlorosilane compound is typically added in an amount of from 1.0 mol% to 5.0 mol%, preferably from 1.1 mol% to 2.5 mol% with respect to the silanol group-containing compound represented by the formula (7A).

In addition, when the reaction is terminated with the chlorosilane compound, hydrogen chloride is generated by the reaction, and hence a base may be added for the purpose of capturing hydrogen chloride. Examples of the base include pyridine, diisopropylamine, triethylamine, and trioctylamine. Of those, triethylamine is preferred from the viewpoint of ease of handling. The base is preferably added in a molar amount equal to or more than that of the chlorosilane compound.

As used herein, the term "(meth)acrylate" means "acrylate or methacrylate," and the same also applies to other similar terms.

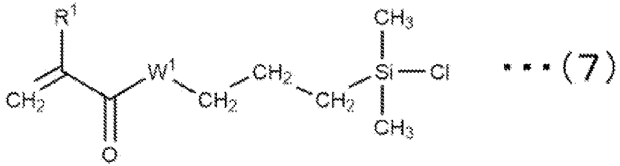

In the formula, R¹ represents H or CH₃, and W¹ represents O or NR⁴, where R⁴ represents H or an alkyl group having 1 to 4 carbon atoms.

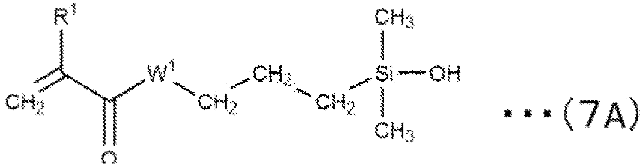

In the formula, R¹ represents H or CH₃, and W¹ represents O or NR⁴, where R⁴ represents H or an alkyl group having 1 to 4 carbon atoms.

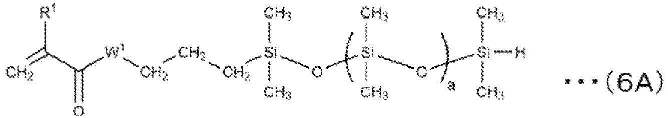

In the formula, R¹ represents H or CH₃, W¹ represents O or NR⁴, where R⁴ represents H or an alkyl group having 1 to 4 carbon atoms, and "a" represents an integer of from 1 to 100.

### <Hydrosilylation Reaction>

The hydrosilylation reaction is a reaction known to a person skilled in the art and refers to a reaction in which a hydrosilane having a silicon-hydrogen bond is added to an unsaturated bond to generate a silicon-carbon bond in the presence of a metal catalyst.

Examples of the metal catalyst include a rhodium catalyst typified by Wilkinson catalyst, and a platinum catalyst typified by a platinum chloride catalyst and Karstedt catalyst. Of those, a platinum chloride catalyst and Karstedt catalyst are preferred.

### <Reaction between Epoxy Group and Carboxylic Acid>

The reaction between an epoxy group and a carboxylic acid is a reaction known to a person skilled in the art and refers to a reaction in which a bond represented by the formula (8) is formed along with ring opening of the epoxy group in the presence of a base catalyst. The base catalyst is not particularly limited, but examples thereof include triethylamine, dimethylamine, diisopropylethylamine, diisopropylamine, sodium hydroxide, and potassium hydroxide. Of those, triethylamine and sodium hydroxide are preferred.

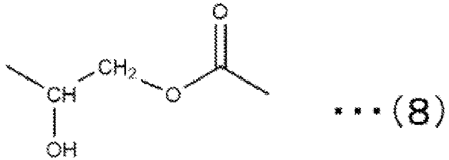

### <Method of producing Compound represented by Formula (1A)>

The compound represented by the formula (1A) is synthesized from the compound represented by the formula (6A) by combining a hydrosilylation reaction and a reaction between an epoxy group and a carboxylic acid. A method of producing the compound represented by the formula (1A) is described below.

The compound represented by the formula (6A) and an excess amount of a vinyl compound having an epoxy group represented by the formula (9) are mixed to perform a hydrosilylation reaction, to thereby provide a compound in which a hydrosilane in the formula (6A) is added to a vinyl group of the formula (9). The unreacted formula (9) compound may be removed by a concentration operation with an evaporator or the like.

Subsequently, an excess amount of a carboxylic acid compound having a phosphorylcholine group represented by the formula (10) is added to perform a reaction between the epoxy group and the carboxylic acid. The reaction between the epoxy group and the carboxylic acid forms the bond represented by the formula (8) to provide a polydimethylsiloxane-containing monomer having a phosphorylcholine group and a hydroxy group represented by the formula (1A). The unreacted carboxylic acid compound having a phosphorylcholine group represented by the formula (10) may be removed by liquid separation purification.

Another example of the method of producing the compound represented by the formula (1A) is the following method.

A vinyl compound having a phosphorylcholine group and a hydroxy group represented by the formula (11) is synthesized by a reaction between the epoxy group of an excess amount of the vinyl compound having an epoxy group represented by the formula (9) and the carboxylic acid of the carboxylic acid compound having a phosphorylcholine group represented by the formula (10). In this case, the unreacted formula (9) compound may be removed by a concentration operation with an evaporator or the like.

Subsequently, the formula (6A) is added to perform a hydrosilylation reaction between the compound represented by the formula (6A) and an excess amount of the vinyl compound having a phosphorylcholine group and a hydroxy group represented by the formula (11). When the hydrosilane in the formula (6A) is added to the vinyl group of the vinyl compound having a phosphorylcholine group and a hydroxy group represented by the formula (11), the polydimethylsiloxane-containing monomer having a phosphorylcholine group and a hydroxy group represented by the formula (1A) is obtained. In this case, the unreacted vinyl compound having a phosphorylcholine group and a hydroxy group represented by the formula (11) may be removed by a liquid separation operation.

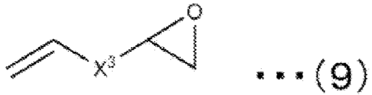

In the formula, X³ represents an alkyl group having 1 to 6 carbon atoms or a divalent group of -R¹²-O-R¹³-, where R¹² represents a divalent alkylene group having 1 to 4 carbon atoms and R¹³ represents a divalent alkylene group having 1 to 6 carbon atoms.

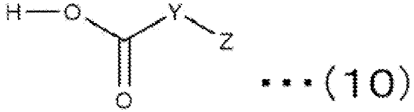

In the formula, Y represents a divalent alkylene group having 1 to 8 carbon atoms or a divalent group of -R⁸-OR⁹-, where R⁸ and R⁹ each independently represent a divalent alkylene group having 1 to 6 carbon atoms, or Y is represented by the formula (3) or the formula (4), and Z represents a phosphorylcholine group.

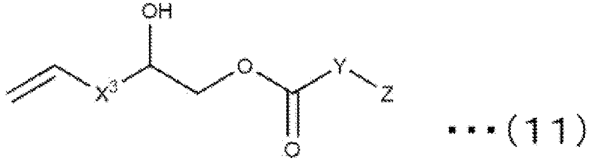

In the formula, X³ represents an alkyl group having 1 to 6 carbon atoms or a divalent group of -R¹²-O-R¹³-, where R¹² represents a divalent alkylene group having 1 to 4 carbon atoms and R¹³ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group.

### <With Regard to Vinyl Compound having Epoxy Group represented by Formula (9)>

The vinyl compound having an epoxy group, which is used for synthesis of the formula (1A), is represented by the formula (9) .

Examples of the compound of the formula (9) include 3,4-epoxy-1-butene, 1,2-epoxy-5-hexene, allyl glycidyl ether, and 1,2-epoxy-9-decene.

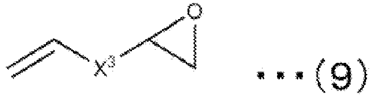

In the formula, X³ represents an alkyl group having 1 to 6 carbon atoms or a divalent group of -R¹²-O-R¹³-, where R¹² represents a divalent alkylene group having 1 to 4 carbon atoms and R¹³ represents a divalent alkylene group having 1 to 6 carbon atoms.

### <With Regard to Carboxylic Acid Compound having Phosphorylcholine Group represented by Formula (10)>

The carboxylic acid compound having a phosphorylcholine group, which is used for synthesis of the formula (1A), is represented by the formula (10).

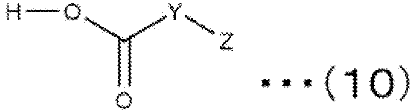

In the formula, Y represents a divalent alkylene group having 1 to 8 carbon atoms or a divalent group of -R⁸-OR⁹-, where R⁸ and R⁹ each independently represent a divalent alkylene group having 1 to 6 carbon atoms, or Y is represented by the formula (3) or the formula (4), and Z represents a phosphorylcholine group.

As the carboxylic acid compound having a phosphorylcholine group represented by the formula (10), for example, in JP 2005-187456 A, there is a description of a compound represented by {in the formula (10), Y: -CH₂-, Z: the formula (5)}.

In addition, as the carboxylic acid compound having a phosphorylcholine group represented by the formula (10), for example, in JP 2017-88530 A, there is a description of a compound represented by {in the formula (10), Y: the formula (3), Z: the formula (5)}.

Further, as the carboxylic acid compound having a phosphorylcholine group represented by the formula (10), for example, in JP 2013-234160 A, there is a description of a compound represented by {in the formula (10), Y: the formula (4), Z: the formula (5)}.

### <With Regard to Vinyl Compound having Phosphorylcholine Group and Hydroxy Group represented by Formula (11)>

The vinyl compound having a phosphorylcholine group and a hydroxy group represented by the formula (11), which is used for synthesis of the formula (1A), is synthesized by a reaction between the epoxy group of the vinyl compound having an epoxy group represented by the formula (9) and the carboxylic acid of the carboxylic acid compound having a phosphorylcholine group represented by the formula (10).

The vinyl compound having a phosphorylcholine group and a hydroxy group represented by the formula (11) is used for synthesis of the polydimethylsiloxane-containing monomer having a phosphorylcholine group and a hydroxy group represented by the formula (1A) by the hydrosilylation reaction with the compound represented by the formula (6A).

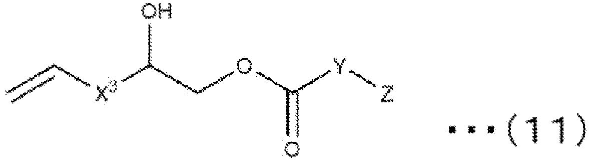

In the formula, X³ represents an alkyl group having 1 to 6 carbon atoms or a divalent group of -R¹²-O-R¹³-, where R¹² represents a divalent alkylene group having 1 to 4 carbon atoms and R¹³ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group.

### <With Regard to Example 1 of Composition of the Present Disclosure>

Example 1 of the composition of the present disclosure includes: at least one kind of polydimethylsiloxane-containing monomer having a phosphorylcholine group and a hydroxy group represented by the formula (1A); and at least one kind of hydrophilic monomer.

The composition of the present disclosure may be polymerized through use of a catalyst or an initiator known to a person skilled in the art.

In Example 1 of the composition of the present disclosure, the content of the polydimethylsiloxane-containing monomer having a phosphorylcholine group and a hydroxy group represented by the formula (1A) is from 10 parts by mass to 60 parts by mass, preferably from 15 parts by mass to 55 parts by mass, still more preferably from 25 parts by mass to 45 parts by mass with respect to the total amount. When the content is less than 10 parts by mass, the transparency of a contact lens may be lowered. When the amount of the polydimethylsiloxane-containing monomer is more than 60 parts by mass, the viscosity of the composition of the present disclosure becomes high, and hence the composition may become difficult to handle.

In Example 1 of the composition of the present disclosure, the content of the hydrophilic monomer is from 20 parts by mass to 80 parts by mass, preferably from 25 parts by mass to 70 parts by mass, more preferably from 30 parts by mass to 60 parts by mass with respect to the total amount. When the content is less than 20 parts by mass, the hydrophilicity of a contact lens may be lowered. When the amount of the hydrophilic monomer is more than 80 parts by mass, sufficient oxygen permeability may not be obtained.

The hydrophilic monomer is a compound having a hydrophilic functional group and a polymerizable vinyl group. The hydrophilic monomer may be selected from the group consisting of, for example: 2-(methacryloyloxyethyl)-2-(trimethylammonioethyl) phosphate; 2-hydroxyethyl (meth)acrylate; N-vinylpyrrolidone; N,N-dimethylacrylamide; 2-hydroxypropyl (meth)acrylate; 2-hydroxybutyl (meth)acrylate; N-methyl-N-vinylacetamide; 2-hydroxyethyl (meth)acrylamide; ethylene glycol monovinyl ether; diethylene glycol monovinyl ether; and mixtures thereof, but is not particularly limited thereto.

### <With Regard to Other Components of Composition>

Example 1 of the composition of the present disclosure may include other components except the polydimethylsiloxane-containing monomer having a phosphorylcholine group and a hydroxy group represented by the formula (1A) and the hydrophilic monomer.

An example of the other components of the composition of the present disclosure is a siloxane monomer. When the siloxane monomer is added to the composition, the oxygen permeability and dynamic characteristics of a polymerization product of the composition can be improved. The siloxane monomer is not particularly limited as long as the siloxane monomer is a compound containing a siloxane and a vinyl group, and examples thereof include 2-hydroxy-3-[3-[methylbis(trimethylsiloxy)silyl]propoxy]propyl methacrylate, 4-(2-hydroxyethyl)=1-[3-tris(trimethylsiloxy)silylpropyl]=2-methylidene succinate, 3-[tris(trimethylsiloxy)silyl]propyl methacrylate, and polydimethylsiloxane monomethacrylate.

The content of the siloxane monomer is typically from 5 parts by mass to 40 parts by mass, preferably from 8 parts by mass to 30 parts by mass, more preferably from 10 parts by mass to 25 parts by mass with respect to the total amount of the monomers.

Another examples of the other components of the composition of the present disclosure is a cross-linking agent. The cross-linking agent is known to a person skilled in the art, and is selected from the group consisting of, for example: tetra(ethylene glycol) di(meth)acrylate; tri(ethylene glycol) di(meth)acrylate; ethylene glycol di(meth)acrylate; di(ethylene glycol) di(meth)acrylate; glycerol dimethacrylate; allyl (meth)acrylate; N,N'-methylenebis(meth)acrylamide; N,N'-ethylenebis(meth)acrylamide; N,N'-dihydroxyethylenebis(meth)acrylamide; triallyl isocyanurate; tetraethylene glycol divinyl ether; triethylene glycol divinyl ether; diethylene glycol divinyl ether; ethylene glycol divinyl ether; trimethylolpropane tri(meth)acrylate; and combinations thereof. The content of the cross-linking agent is typically from 0.1 part by mass to 5 parts by mass, preferably from 0.3 part by mass to 3 parts by mass, more preferably from 0.5 part by mass to 2 parts by mass with respect to the total amount of the composition.

In Example 1 of the composition of the present disclosure, when the content of the polydimethylsiloxane-containing monomer having a phosphorylcholine group and a hydroxy group represented by the formula (1A) is set to 100 parts by mass, the content of one or more kinds of hydrophilic monomers may be set to from 33 parts by mass to 800 parts by mass and the content of one or more kinds of siloxane monomers may be set to from 8 parts by mass to 400 parts by mass.

### <With Regard to Example 2 of Composition of the Present Disclosure>

Example 2 of the composition of the present disclosure includes: at least one kind of polydimethylsiloxane-containing monomer having a phosphorylcholine group and a hydroxy group represented by the formula (1A); at least one kind of hydrophilic monomer; and at least one kind of hydrophilic polymer.

Alternatively, Example 2 of the composition of the present disclosure includes: at least one kind of polydimethylsiloxane-containing monomer having a phosphorylcholine group and a hydroxy group represented by the formula (1A); at least one kind of hydrophilic monomer; at least one kind of hydrophilic polymer; and at least one kind of hydroxy group-containing siloxane monomer.

Example 2 of the composition of the present disclosure may be polymerized through use of a catalyst or an initiator known to a person skilled in the art.

In Example 2 of the composition of the present disclosure, the content of the polydimethylsiloxane-containing monomer having a phosphorylcholine group and a hydroxy group represented by the formula (1A) is from 5 parts by mass to 50 parts by mass, preferably from 10 parts by mass to 40 parts by mass, more preferably from 15 parts by mass to 35 parts by mass with respect to the total amount of the monomers. When the content is less than 5 parts by mass, the transparency of a polymerization product obtained by polymerization is lowered. When the content is more than 50 parts by mass, the surface wettability of the polymerization product is lowered.

In Example 2 of the composition of the present disclosure, the content of the hydrophilic monomer is typically from 20 parts by mass to 90 parts by mass, preferably from 40 parts by mass to 80 parts by mass with respect to the total amount of the monomers.

In Example 2 of the composition of the present disclosure, the content of the hydroxy group-containing siloxane monomer is typically from 20 parts by mass to 90 parts by mass, preferably from 40 parts by mass to 80 parts by mass with respect to the total amount of the monomers.

In Example 2 of the composition of the present disclosure, the content of the hydrophilic polymer is from 0.1 part by mass to 15 parts by mass, preferably from 1 part by mass to 10 parts by mass with respect to the total amount of the composition.

### <With Regard to Hydrophilic Monomer>

The hydrophilic monomer is a compound having a hydrophilic functional group and a polymerizable vinyl group. The hydrophilic monomer may be selected from the group consisting of, for example: 2-hydroxyethyl (meth)acrylate; N-vinylpyrrolidone; N,N-dimethylacrylamide; 2-(methacryloyloxyethyl)-2-(trimethylammonioethyl) phosphate; 2-hydroxypropyl (meth)acrylate; 2-hydroxybutyl (meth)acrylate; N-methyl-N-vinylacetamide; and mixtures thereof, but is not particularly limited thereto.

### <With Regard to Hydrophilic Polymer>

Examples of the hydrophilic polymer may include polyamide, polylactam, polyimide, and polylactone. Those hydrophilic polymers are each preferably a hydrogen bond acceptor that effectively becomes more hydrophilic through a hydrogen bond to water in an aqueous environment.

The hydrophilic polymer is preferably a linear polymer having a cyclic moiety in a polymer main chain thereof. The cyclic moiety is more preferably a cyclic moiety in a cyclic amide or imide. A polymer of this kind preferably includes, for example, polyvinylpyrrolidone and polyvinylimidazole, but a polymer such as polydimethylacrylamide is also useful in the ability thereof. Polyvinylpyrrolidone is the most preferred hydrophilic polymer.

The molecular weight of the hydrophilic polymer is not particularly limited, but is generally from 100,000 to 500,000, more preferably from 300,000 to 500,000.

### <With Regard to Other Components of Composition>

Example 2 of the composition of the present disclosure may include other components except the polydimethylsiloxane-containing monomer having a phosphorylcholine group and a hydroxy group represented by the formula (1A), the hydrophilic monomer, and the hydrophilic polymer.

An example of the other components of Example 2 of the composition of the present disclosure is a siloxane monomer. When the siloxane monomer is added to the composition, the oxygen permeability and dynamic characteristics of a polymerization product of the composition can be improved. The siloxane monomer is not particularly limited as long as the siloxane monomer is a compound containing a siloxane and a vinyl group, but is preferably a hydroxy group-containing siloxane monomer from the viewpoint of compatibility with the composition. The hydroxy group-containing siloxane monomer is a compound containing a siloxane, a vinyl group, and a hydroxy group, and examples thereof include 2-hydroxy-3-[3-[methylbis(trimethylsiloxy)silyl]propoxy]propyl methacrylate and 4-(2-hydroxyethyl)=1-[3-tris(trimethylsiloxy)silylpropyl]=2-methylidene succinate.

The content of the siloxane monomer or the hydroxy group-containing siloxane monomer is typically from 5 parts by mass to 50 parts by mass, preferably from 10 parts by mass to 40 parts by mass, more preferably from 15 parts by mass to 30 parts by mass with respect to the total amount of the monomers.

Another example of the other components of Example 2 of the composition of the present disclosure is a cross-linking agent. The cross-linking agent is known to a person skilled in the art, and for example, is selected from the group consisting of: tetra(ethylene glycol) di(meth)acrylate; tri(ethylene glycol) di(meth)acrylate; ethylene glycol di(meth)acrylate; di(ethylene glycol) di(meth)acrylate; glycerol dimethacrylate; allyl (meth)acrylate; N,N'-methylenebis(meth)acrylamide; N,N'-ethylenebis(meth)acrylamide; N,N'-dihydroxyethylenebis(meth)acrylamide; triallyl isocyanurate; tetraethylene glycol divinyl ether; triethylene glycol divinyl ether; diethylene glycol divinyl ether; ethylene glycol divinyl ether; and combinations thereof. The content of the cross-linking agent is typically from 0.1 part by mass to 5 parts by mass, preferably from 0.3 part by mass to 3 parts by mass, more preferably from 0.5 part by mass to 2 parts by mass with respect to the total amount of the composition.

In Example 2 of the composition of the present disclosure, when the content of the polydimethylsiloxane-containing monomer having a phosphorylcholine group and a hydroxy group represented by the formula (1A) is set to 100 parts by mass, the content of one or more kinds of hydrophilic monomers may be set to from 40 parts by mass to 1,800 parts by mass, the content of one or more kinds of hydrophilic polymers may be set to from 0.2 part by mass to 300 parts by mass, and the content of one or more kinds of siloxane monomers may be set to from 40 parts by mass to 1,800 parts by mass.

The combination of the polydimethylsiloxane-containing monomer having a phosphorylcholine group and a hydroxy group represented by the formula (1A), the hydrophilic monomer, and the hydrophilic polymer in Example 2 of the composition of the present disclosure is not particularly limited, but preferred combinations are, for example, as described below.

The polydimethylsiloxane-containing monomer having a phosphorylcholine group and a hydroxy group represented by the formula (1A), N,N-dimethylacrylamide (DMAA), 2-hydroxybutyl methacrylate (HBMA), N-vinylpyrrolidone (NVP), and polyvinylpyrrolidone (PVP)

The polydimethylsiloxane-containing monomer having a phosphorylcholine group and a hydroxy group represented by the formula (1A), DMAA, 2-hydroxyethyl methacrylate (HEMA), NVP, and PVP

The polydimethylsiloxane-containing monomer having a phosphorylcholine group and a hydroxy group represented by the formula (1A), DMAA, HBMA, NVP, 2-(methacryloyloxyethyl)-2-(trimethylammonioethyl) phosphate (MPC), and PVP

The polydimethylsiloxane-containing monomer having a phosphorylcholine group and a hydroxy group represented by the formula (1A), DMAA, 2-hydroxypropyl methacrylate (HPMA), NVP, and PVP

The polydimethylsiloxane-containing monomer having a phosphorylcholine group and a hydroxy group represented by the formula (1A), DMAA, HPMA, NVP, N-methyl-N-vinylacetamide (MVA), and PVP

The polydimethylsiloxane-containing monomer having a phosphorylcholine group and a hydroxy group represented by the formula (1A), DMAA, HBMA, NVP, and 4-(2-hydroxyethyl)=1-[3-tris(trimethylsiloxy)silylpropyl]=2-methylidene succinate (ETS)

The polydimethylsiloxane-containing monomer having a phosphorylcholine group and a hydroxy group represented by the formula (1A), DMAA, HEMA, NVP, and ETS

The polydimethylsiloxane-containing monomer having a phosphorylcholine group and a hydroxy group represented by the formula (1A), DMAA, HBMA, NVP, MPC, and 2-hydroxy-3-[3-[methylbis(trimethylsiloxy)silyl]propoxy]propyl methacrylate (SiGMA)

The polydimethylsiloxane-containing monomer having a phosphorylcholine group and a hydroxy group represented by the formula (1A), DMAA, HPMA, NVP, and ETS

The polydimethylsiloxane-containing monomer having a phosphorylcholine group and a hydroxy group represented by the formula (1A), DMAA, HPMA, NVP, MVA, and ETS

The combination of the polydimethylsiloxane-containing monomer having a phosphorylcholine group and a hydroxy group represented by the formula (1A), the siloxane monomer, and the hydrophilic monomer in Example 1 of the composition of the present disclosure is not particularly limited, but preferred combinations are, for example, as described below.

The polydimethylsiloxane-containing monomer having a phosphorylcholine group and a hydroxy group represented by the formula (1A), 3-[tris(trimethylsiloxy)silyl]propyl methacrylate (TRIS), MPC, HBMA, and NVP

The polydimethylsiloxane-containing monomer having a phosphorylcholine group and a hydroxy group represented by the formula (1A), ETS, MPC, HEMA, and NVP

The polydimethylsiloxane-containing monomer having a phosphorylcholine group and a hydroxy group represented by the formula (1A), ETS, MPC, HBMA, and NVP

The polydimethylsiloxane-containing monomer having a phosphorylcholine group and a hydroxy group represented by the formula (1A), ETS, MPC, HBMA, and NVP

The polydimethylsiloxane-containing monomer having a phosphorylcholine group and a hydroxy group represented by the formula (1A), monomethacryloxypropyl terminated polydimethylsiloxane (MCR-M11), SiGMA, MPC, HBMA, and NVP

The polydimethylsiloxane-containing monomer having a phosphorylcholine group and a hydroxy group represented by the formula (1A), TRIS, MPC, HEMA, and NVP

The polydimethylsiloxane-containing monomer having a phosphorylcholine group and a hydroxy group represented by the formula (1A), SiGMA, MPC, HBMA, and NVP

The polydimethylsiloxane-containing monomer having a phosphorylcholine group and a hydroxy group represented by the formula (1A), SiGMA, MPC, HEMA, and NVP

The polydimethylsiloxane-containing monomer having a phosphorylcholine group and a hydroxy group represented by the formula (1A), MCR-M11, SiGMA, MPC, HEMA, and NVP

The polydimethylsiloxane-containing monomer having a phosphorylcholine group and a hydroxy group represented by the formula (1A), MCR-M11, MPC, HBMA, and NVP

The polydimethylsiloxane-containing monomer having a phosphorylcholine group and a hydroxy group represented by the formula (1A), MCR-M11, MPC, HEMA, and NVP

### <Configuration Examples of Compound represented by Formula (1A) of the Present Disclosure>

Configuration examples of the compound represented by the formula (1A) of the present disclosure are shown in Table 1 below, but are not particularly limited thereto.

In addition, R¹, W¹, "b", "c", and X¹, Y, and Z of A⁰ shown in Table 1 may be each changed to other combinations to form the compound represented by the formula (1A) of the present disclosure.

### <With Regard to Polymerization Product of the Present Disclosure>

A polymerization product of the present disclosure may be obtained by polymerizing the composition of the present disclosure, and in the polymerization, any solvent and any thermal initiator or photoinitiator serving as a radical initiator for polymerization may be used.

Further, a polymerization method is not particularly limited, and various methods well-known to a person skilled in the art may be used. For example, the polymerization may be performed by a known method including mixing and uniformly dissolving the composition, appropriately adding a thermal polymerization initiator or a photopolymerization initiator typified by a peroxide or an azo compound, and dispensing the resultant into a contact lens mold, followed by heating, UV irradiation, or the like. The polymerization may be performed in the atmosphere, but may be performed under an atmosphere of an inert gas, such as nitrogen or argon, for the purpose of improving a polymerization rate. When the polymerization is performed under an atmosphere of an inert gas, the pressure in a polymerization system is desirably set to 1 kgf/cm² or less.

When the solvent is used, suitable examples of the solvent include, but not limited to, methanol, ethanol, 1-propanol, 2-propanol, 1-butanol, 2-butanol, tert-butanol, 1-pentanol, 2-pentanol, tert-amyl alcohol, 1-hexanol, 1-octanol, 1-decanol, 1-dodecanol, glycolic acid, lactic acid, and acetic acid. Those solvents may be used alone or as a mixture thereof. One or more kinds selected from the group consisting of: ethanol; 1-propanol; 2-propanol; and 1-hexanol are preferred from the viewpoints of availability and pH stability.

In Example 1 of the composition of the present disclosure, it is more preferred that the solvent be free from being blended.

A suitable thermal polymerization initiator is known to a person skilled in the art, and examples thereof include a peroxide, a hydroperoxide, an azo-bis(alkyl- or cycloalkylnitrile), a persulfate, a percarbonate, and mixtures thereof. Examples thereof include benzoyl peroxide, tert-butyl peroxide, di-tert-butyl-diperoxyphthalate, tert-butyl hydroperoxide, azo-bis(isobutyronitrile) (AIBN), 1,1-azodiisobutylamidine, 1,1'-azo-bis(1-cyclohexanecarbonitrile), and 2,2'-azo-bis(2,4-dimethylvaleronitrile). The polymerization is conveniently performed at a high temperature, such as a temperature of from 25°C to 140°C, preferably from 40°C to 120°C in the above-mentioned solvent. A reaction time may vary within a wide limit, but is conveniently, for example, from 1 hour to 24 hours or preferably from 1.5 hours to 12 hours. It is advantageous that the components and the solvent to be used in the polymerization reaction be degassed in advance and the above-mentioned copolymerization reaction be performed under an inert atmosphere, for example, under a nitrogen or argon atmosphere.

Suitable examples of the photoinitiator include benzoin methyl ether, diethoxyacetophenone, benzoylphosphine oxide, and 1-hydroxycyclohexyl phenyl ketone. Of those, Darocur (Registered trademark) 1173 and Darocur (Registered trademark) 2959, Irgacure (Registered trademark) 819, and a germanium-based Norrish-type I photoinitiator are preferred. Examples of the benzoylphosphine oxide initiator include 2,4,6-trimethylbenzoyldiphenylphosphine oxide, bis-(2,6-dichlorobenzoyl)-4-N-propylphenylphosphine oxide, and bis-(2,6-dichlorobenzoyl)-4-N-butylphenylphosphine oxide. For example, a reactive photoinitiator that may be incorporated into a macromer or may be used as a special monomer is also suitable.

After the polymerization, the polymerization product may be released from the mold by a known method and taken out in a dry state. Alternatively, the polymerization product may also be released by immersing the polymerization product in a solvent (e.g., water, ethanol, 1-propanol, 2-propanol, and a mixed solution thereof) together with the mold to swell the polymerization product. Further, the resultant may be washed by being repeatedly immersed in those solvents to remove residues of each of components, residual materials, by-products, and the like, to thereby provide a polymerization product.

Examples of the solvent to be used for washing include water, ethanol, 1-propanol, 2-propanol, and mixtures thereof. For washing, for example, the polymerization product may be immersed in an alcohol-based solvent at a temperature of from 10°C to 40°C for from 10 minutes to 10 hours. In addition, after the washing with an alcohol-based solvent, the resultant may be washed by being immersed in an aqueous solution having an alcohol concentration of from 20 mass% to 50 mass% for from 10 minutes to 10 hours.

Any solvent may be used as the solvent to be used for washing as long as the solvent has quality for drugs, quasi-drugs, and medical devices.

### <With Regard to Ophthalmic Device of the Present Disclosure>

An ophthalmic device of the present disclosure is substantially formed of the polymerization product of the present disclosure or includes the polymerization product of the present disclosure. The ophthalmic device in the present disclosure encompasses a contact lens, a soft contact lens, a hard contact lens, an intraocular lens, and an artificial cornea, but is not particularly limited thereto.

### Examples

The present disclosure is described in more detail below by way of Examples and Comparative Examples. However, the present disclosure is not limited thereto.

Conditions for ¹H NMR analysis performed in Examples are shown below.
Measurement apparatus: JNM-AL400 manufactured by JEOL Ltd.
Solvent: DMSO-d6
Relaxation time: 15 seconds
Number of scans: 32 times

### <Synthesis of Polydimethylsiloxane-containing Monomer having Phosphorylcholine Group and Hydroxy Group>

### [Synthesis Example 1-1] Synthesis of Compound represented by Formula (6A)

10.8 g (49.4 mmol) of 3-(chlorodimethylsilyl)propyl methacrylate was dissolved in 43.6 g of tetrahydrofuran, and 2.36 g (98.5 mmol) of lithium hydroxide was added. After an elapse of 30 minutes, 43.6 g of heptane was added, and pressure filtration was performed to remove insoluble matter. (As a result of the reaction, a silanol group-containing compound having a vinyl group was obtained.)

71.2 g of tetrahydrofuran was added to a filtrate of the pressure filtration, and 22.0 g (98.8 mmol) {amount equivalent to a=6 in the formula (6A)} of hexamethylcyclotrisiloxane was added. Subsequently, the entire amount of a catalyst solution (prepared in a separate flask by dissolving 0.74 g of diazabicycloundecene in 7.52 g of tetrahydrofuran) was added. After an elapse of 4 hours, 6.60 g of triethylamine was added, 5.14 g of chlorodimethylsilane was added, and the reaction was terminated.

The solution after the reaction was concentrated with an evaporator, diluted with heptane, and then washed with ion-exchanged water. The heptane layer was recovered, dehydrated with sodium sulfate, and finally concentrated with an evaporator to provide 26.2 g of a compound represented by the formula (6A) {R¹: -CH₃, W¹: -O-, a: 6}.

### [Synthesis Examples 1-2 to 1-4] Synthesis of Compounds each represented by Formula (6A)

The formula (6A) compounds of Synthesis Examples 1-2 to 1-4 were each obtained as described below by the same method as that in Synthesis Example 1-1 except that the loading amount of hexamethylcyclotrisiloxane was changed.
Synthesis Example 1-2: the formula (6A) {R¹: -CH₃, W¹: -O-, a: 12}
Synthesis Example 1-3: the formula (6A) {R¹: -CH₃, W¹: -O-, a: 19}
Synthesis Example 1-4: the formula (6A) {R¹: -CH₃, W¹: -O-, a: 48}

### [Example 1-1] Synthesis of Compound represented by Formula (1A)

After 3.00 g of the compound represented by the formula (6A) {R¹: -CH₃, W¹: -O-, a: 6} obtained in Synthesis Example 1-1 and 0.81 g of allyl glycidyl ether were dissolved in 7.07 g of toluene, 18 µL of Karstedt catalyst (manufactured by Tokyo Chemical Industry Co., Ltd.) adjusted to a 10 wt% xylene solution was added to perform a hydrosilylation reaction overnight. 0.01 g of activated carbon was added, and the mixture was stirred for 30 minutes. The activated carbon was removed by pressure filtration, and the filtrate was concentrated with an evaporator.

Subsequently, 2.87 g of the formula (10) {in the formula (10), Y: the formula (3), Z: the formula (5)} compound of this description, which is described also in JP 2017-88530 A, was added. Then, 5.31 g of methanol, 5.31 g of 2-propanol, and 0.29 g of triethylamine were added. After that, the temperature of the mixture was raised to perform a reaction (reaction between an epoxy group and a carboxylic acid) overnight in a reflux state.

After the reaction, an insoluble portion was removed by pressure filtration, and the filtrate was concentrated with an evaporator. The residue was mixed with 20.0 g of ion-exchanged water, 6.68 g of 2-propanol, and 20.0 g of heptane, followed by stirring. After being left to stand still, the mixture was separated into two layers, and the lower layer was discarded. After that, the mixing with ion-exchanged water, 2-propanol, and heptane, the stirring, and the discarding of the lower layer were repeated twice, and the remaining layer was concentrated with an evaporator to provide 4.35 g of a transparent viscous material.

It was recognized from ¹H NMR analysis that a compound was represented by the formula (1A) {R¹: -CH₃, W¹: -O-, a: 6, in A⁰, X¹: -CH₂CH₂CH₂-O-CH₂-, Y: the formula (3), Z: the formula (5)} was obtained. The synthesis results of Example 1-1 are shown in Table 1.

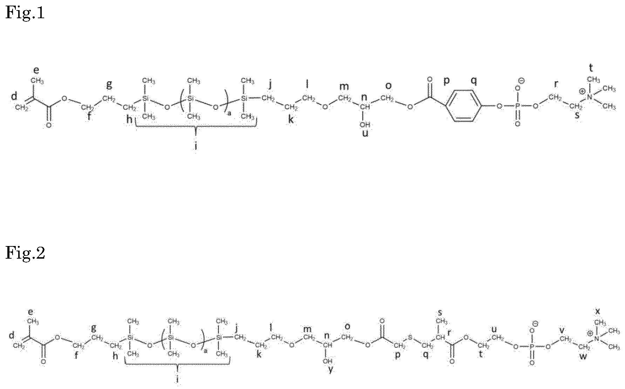

The attribution results of the ¹H NMR analysis of Example 1-1 are shown below (FIG. 1).

¹H NMR (DMSO-d6): δ=0.0 ppm (i), 0.5 ppm (h, j), 1.6 ppm to 1.7 ppm (g, k), 1.9 ppm (e), 3.2 ppm (t), 3.3 ppm to 3.8 ppm (l, m, o, s), 4.0 ppm to 4.5 ppm (f, n, r), 5.6 ppm, 6.1 ppm (d), 5.8 ppm (u), 7.3 ppm (q), 8.0 ppm (p)

### [Example 1-2] Synthesis of Compound represented by Formula (1A)

After 3.67 g of the formula (10) {in the formula (10), Y: the formula (4), R³: -CH₃, X²: -CH₃, Z: the formula (5)} compound of this description, which is described also in JP 2013-234160 A, 1.21 g of allyl glycidyl ether, 3.76 g of methanol, 3.76 g of 2-propanol, and 0.29 g of triethylamine were added, the temperature of the mixture was raised to perform a reaction overnight in a reflux state. After the reaction, an insoluble portion was removed by pressure filtration, and the filtrate was concentrated with an evaporator.

Subsequently, 3.00 g of the compound represented by the formula (6A) obtained in Synthesis Example 1-1, 7.02 g of 2-propanol, and 72 µL of a platinum chloride catalyst adjusted to a 4 wt% 2-propanol solution were added to perform a reaction overnight. 0.05 g of activated carbon was added, and the mixture was stirred for 30 minutes. The activated carbon was removed by pressure filtration, and the filtrate was concentrated with an evaporator.

The residue was mixed with 21.4 g of ion-exchanged water, 7.12 g of 2-propanol and 21.4 g of heptane, followed by stirring. After being left to stand still, the mixture was separated into two layers, and the lower layer was discarded. After that, the mixing with ion-exchanged water, 2-propanol, and heptane, the stirring, and the discarding of the lower layer were repeated twice, and the remaining layer was concentrated with an evaporator to provide 4.57 g of a transparent viscous material.

It was recognized from ¹H NMR analysis that a compound represented by the formula (1A) {R¹: -CH₃, W¹: -O-, a: 6, in A⁰, X¹: -CH₂CH₂CH₂-O-CH₂-, Y: the formula (4), R³: -CH₃, X²: -CH₃, Z: the formula (5)} was obtained. The synthesis results of Example 1-2 are shown in Table 1.

The attribution results of the ¹H NMR analysis of Example 1-2 are shown below (FIG. 2).

¹H NMR (DMSO-d6): δ=0.0 ppm (i), 0.5 ppm to 0.6 ppm (h, j), 1.2 ppm (s), 1.6 ppm to 1.7 ppm (g, k), 1.9 ppm (e), 2.6 ppm to 2.9 ppm (q, r), 3.2 ppm (x), 3.3 ppm to 3.8 ppm (l, m, o, p, w), 3.9 ppm to 4.7 ppm (f, n, t, u, v), 5.6 ppm, 6.1 ppm (d), 5.8 ppm (y)

### [Example 1-3] Synthesis of Compound represented by Formula (1A)

The carboxylic acid compound having a phosphorylcholine group represented by the formula (10) was subjected to a synthesis reaction through use of the formula (10) {Y: -CH₂-, Z: the formula (5)}, which is described in JP 2005-187956 A, by the same method as that in Example 1-1 to provide the formula (1A) {R¹: -CH₃, W¹: -O-, a: 6; in A⁰, X¹: -CH₂CH₂CH₂-O-CH₂-, Y: -CH₂-, Z: the formula (5)}. The synthesis results of Example 1-3 are shown in Table 1.

### [Example 1-4] Synthesis of Compound represented by Formula (1A)

A reaction was similarly performed through use of the formula (6A) compound obtained in Synthesis Example 1-2 and 1,2-epoxy-5-hexene instead of allyl glycidyl ether by the same method as that in Example 1-1 to provide the formula (1A) {R¹: -CH₃, W¹: -O-, a: 12; in A⁰, X¹: -CH₂CH₂CH₂CH₂-, Y: the formula (3), Z: the formula (5)}. The synthesis results of Example 1-4 are shown in Table 1.

### [Example 1-5] Synthesis of Compound represented by Formula (1A)

A reaction was similarly performed through use of the formula (6A) compound obtained in Synthesis Example 1-3 by the same method as that in Example 1-1 to provide the formula (1A) {R¹: -CH₃, W¹: -O-, a: 19; in A⁰, X¹: -CH₂CH₂CH₂-O-CH₂-, Y: the formula (3), Z: the formula (5)}. The synthesis results of Example 1-5 are shown in Table 1.

### [Example 1-6] Synthesis of Compound represented by Formula (1A)

A reaction was similarly performed through use of the formula (6A) compound obtained in Synthesis Example 1-4 by the same method as that in Example 1-1 to provide the formula (1A) {R¹: -CH₃, W¹: -O-, a: 48; in A⁰, X¹: -CH₂CH₂CH₂-O-CH₂-, Y: the formula (3), Z: the formula (5)}. The synthesis results of Example 1-6 are shown in Table 1.

### [Synthesis Example 1-5] Preparation of Formula (1A) Comparative Compound

MCR-M11 {monomethacryloxypropyl terminated polydimethylsiloxane (molecular weight≈900) manufactured by Gelest Inc.)} was prepared as a formula (1A) comparative compound. The prepared formula (1A) comparative compound was a compound, which corresponded to R¹: -CH₃, W¹: -O-, a: about 9 in the formula (1A) and in which an A⁰ portion was substituted by -CH₂CH₂CH₂CH₃, as described in a product catalog.

### [Synthesis Example 1-6] Synthesis of Formula (1A) Comparative Compound

After 3.00 g of the compound represented by the formula (6A) obtained in Synthesis Example 1-3 and 0.24 g of 1-hexene were dissolved in 6.45 g of toluene, 10 µL of Karstedt catalyst (manufactured by Tokyo Chemical Industry Co., Ltd.) adjusted to a 10 wt% xylene solution was added to perform a reaction overnight. 0.01 g of activated carbon was added, and the mixture was stirred for 30 minutes. The activated carbon was removed by pressure filtration. Thus, 3.01 g of a formula (1A) comparative compound which corresponded to R¹: -CH₃, W¹: -O-, a: 19 in the formula (1A) and in which an A⁰ portion was substituted by - CH₂CH₂CH₂CH₂CH₂CH₃, was obtained. The synthesis results of Synthesis Example 1-6 are shown in Table 1.

### [Synthesis Example 1-7] Synthesis of Formula (1A) Comparative Compound

After 3.00 g of the compound represented by the formula (6A) obtained in Synthesis Example 1-2 and 0.47 g of 3-buten-1-ol were dissolved in 6.45 g of toluene, 10 µL of Karstedt catalyst (manufactured by Tokyo Chemical Industry Co., Ltd.) adjusted to a 10 wt% xylene solution was added to perform a reaction overnight. 0.01 g of activated carbon was added, and the mixture was stirred for 30 minutes. The activated carbon was removed by pressure filtration. Thus, 3.12 g of a formula (1A) comparative compound which corresponded to R¹: -CH₃, W¹: - O-, a: 12 in the formula (1A) and in which an A⁰ portion was substituted by -CH₂CH₂CH₂CH₂OH, was obtained. The synthesis results of Synthesis Example 1-7 are shown in Table 1.

**Table 1**

| | | Example 1-1 | Example 1-2 | Example 1-3 | Example 1-4 | Example 1-5 | Example 1-6 |
|---|---|---|---|---|---|---|---|
| R¹ | | -CH₃ | -CH₃ | -CH₃ | -CH₃ | -CH₃ | -CH₃ |
| W¹ | | -O- | -O- | -O- | -O- | -O- | -O- |
| a | | 6 | 6 | 6 | 12 | 19 | 48 |
| A⁰ | X¹ | CH₂CH₂CH₂-O-CH₂- | -CH₂CH₂CH₂-O-CH₂- | -CH₂CH₂CH₂-O-CH₂- | CH₂CH₂CH₂C H2- | CH₂CH₂CH ₂-O-CH₂- | CH₂CH₂CH ₂-O-CH₂- |
| | Y | Formula (3) | Formula (4) R³: -CH₃. X²: -CH₃ | -CH₂- | Formula (3) | Formula (3) | Formula (3) |
| | Z | Formula (5) | Formula (5) | Formula (5) | Formula (5) | Formula (5) | Formula (5) |

| Formula (1A) comparative compound | Synthesis Example 1-5 | Synthesis Example 1-6 | Synthesis Example 1-7 |
|---|---|---|---|
| R¹ | -CH₃ | -CH₃ | -CH₃ |
| W¹ | -O- | -O- | -O- |
| a | 9 | 19 | 12 |
| Portion correspond! ng to A⁰ | - CH₂CH₂CH₂C H₃ | - CH₂CH₂CH₂CH₂CH₂ CH₃ | - CH₂CH₂CH₂CH₂ OH |

### <Polymerization of Composition>

### ∘Components used in Example 2 and Comparative Example 2

The components of compositions used in Examples 2 and Comparative Examples except the polydimethylsiloxane-containing monomer having a phosphorylcholine group and a hydroxy group and the comparative compound thereof are described below.

### · Hydrophilic Monomer

DMAA: N,N-dimethylacrylamide
HEMA: 2-hydroxyethyl methacrylate
HPMA: 2-hydroxypropyl methacrylate (mixture of 2-hydroxypropyl ester and 2-hydroxy-1-methylethyl ester)
HBMA: 2-hydroxybutyl methacrylate
NVP: N-vinylpyrrolidone
MVA: N-methyl-N-vinylacetamide
MPC: 2-(methacryloyloxyethyl)-2-(trimethylammonioethyl) phosphate

### · Hydroxy Group-containing Siloxane Monomer

ETS: 4-(2-hydroxyethyl)=1-[3-tris(trimethylsiloxy)silylpropyl]=2-methylidene succinate
SiGMA: 2-hydroxy-3-[3-[methylbis(trimethylsiloxy)silyl]propoxy]propyl methacrylate

### -Hydrophilic Polymer

PVP K90: polyvinylpyrrolidone K90 (manufactured by Fujifilm Wako Pure Chemical Corporation)

### · Cross-linking Agent

TEGDMA: tetraethylene glycol dimethacrylate

### -Solvent

HexOH: 1-hexanol

### -Initiator

AIBN: 2,2'-azobis(isobutyronitrile)

### ∘Evaluation Method

Evaluation methods in Examples 2 and Comparative Examples 2 are as described below.

### [Evaluation of Composition Uniformity]

The uniformity of a composition before polymerization was evaluated by the following method.

The prepared composition was placed in a colorless and transparent container, and was visually evaluated by giving a score in accordance with the following criteria.
1: The composition is uniform and transparent.
2: The composition is non-uniform, or white turbidity, a precipitate, or the like is observed.

The composition given the score of "1" was subjected to a polymerization step, and was evaluated for its polymerization product transparency and anti-lipid adhesion property. The composition given the score of "2" was not subjected to the subsequent polymerization step and evaluation for its polymerization product transparency and anti-lipid adhesion property.

### [Evaluation of Polymerization Product Transparency]

The transparency of a polymerization product obtained by polymerizing a composition was evaluated by the following method.

The polymerization product was immersed in physiological saline overnight, and in a windless room, the polymerization product was taken out from the physiological saline. The polymerization product was exposed to illumination, was visually observed for its appearance, and was evaluated by giving a score in accordance with the following criteria.
1: The polymerization product is transparent without turbidity.
2: The polymerization product is slightly white-turbid.
3: The polymerization product is somewhat white-turbid and semi-transparent.
4: The polymerization product is white-turbid and is not transparent at all.
5: The polymerization product is completely white.

### [Evaluation of Anti-lipid Adhesion Property]

The anti-lipid adhesion property of a polymerization product produced in each of Examples was evaluated by the following procedure.

First, artificial lipid was prepared by the method described below. Subsequently, after the polymerization product immersed overnight in physiological saline was immersed in 4 mL of the artificial lipid for 4 hours, the polymerization product was rinsed lightly with physiological saline, and moisture was removed. The polymerization product was visually observed for its appearance, and was evaluated by giving a score in accordance with the following criteria.
1: The polymerization product is transparent without white turbidity.
2: The polymerization product is slightly white-turbid.
3: The polymerization product is partially white-turbid.
4: The polymerization product is mostly white-turbid.
5: The polymerization product is white-turbid in its entirety.

### · Preparation of Artificial Lipid

1) 0.5 g of mixed lipid having composition described below was mixed with 100 mL of a phosphate-borate buffer solution described below.
2) The mixture was suspended with a homomixer at 60°C.
3) The mixture was adjusted to a pH of 7.0 with 1 N hydrochloric acid.

### Composition of Mixed Lipid

| | |
|---|---|
| Oleic acid | 0.06 g |
| Linolenic acid | 0.06 g |
| Palmitic acid | 0.06 g |
| Tripalmitic acid | 0.81 g |
| Cetyl alcohol | 0.20 g |
| Cetyl myristate | 0.81 g |
| Cholesterol | 0.08 g |
| Cholesterol palmitate | 0.08 g |
| Lecithin (derived from egg) | 2.83 g |

### Composition of Phosphate-Borate Buffer Solution

| | |
|---|---|
| Sodium chloride | 2.25 g |
| Potassium dihydrogen phosphate | 1.25 g |
| Sodium tetraborate decahydrate | 5.65 g |

### Ion-exchanged water Total amount is set to 250 mL

Reference: Kaoru Iwai; Mari Moriyama; Masaki Imayasu; Hidenari Tanaka: Lipid Adsorption to Contact Lens Materials., Journal of Japan Contact Lens Society, 1995, 37(1), 58-61.

### [Evaluation of Wettability]

The wettability of a contact lens was evaluated by a waterfilm break up time (WBUT). A contact lens-shaped polymerization product of each of Examples was slowly taken out from the physiological saline through use of tweezers or the like, and the time until a water film was taken off was measured. The measured time was defined as a WBUT, and was evaluated by giving a score in accordance with the following criteria.
1: 10 seconds or more
2: 5 seconds or more and less than 10 seconds
3: less than 5 seconds

### [Evaluation of Antifouling Property]

As the evaluation of an antifouling property, coatability was evaluated in accordance with the procedure described below.

### <Evaluation Method for Coatability>

0.05 g of Sudan Black B was dissolved in 10 g of tocopherol, and 40 g of liquid paraffin was added. The resultant was uniformly mixed to provide a dyeing solution. The contact lens-shaped polymerization product of each of Examples was taken out from a glass vial, and moisture on the surface of the lens was wiped off. Then, the polymerization product was immersed in 1 mL of the dyeing solution for 5 minutes. The polymerization product was taken out and an excess of the dyeing solution was removed with physiological saline and a clean cloth wetted with physiological saline. The contact lens-shaped polymerization product of Example dyed with the dyeing solution was immersed in 1.5 mL of physiological saline, and was measured for its absorbance at 600 nm. The absorbance of the contact lens-shaped polymerization product of Example before dyeing was represented by A₀, and the absorbance of the contact lens-shaped polymerization product of Example after dyeing was represented by A₁.

An absorbance increased by the dyeing was calculated in accordance with the following equation (1), and was evaluated by giving a score in accordance with the following criteria. Absorbance=A₁-A₀···Equation (A)
1: less than 0.05
2: 0.05 or more and less than 0.10
3: 0.10 or more and less than 0.15
4: 0.15 or more and less than 0.2
5: 0.2 or more

### [Example 2-1]

20.0 Parts by mass of the formula (1A) compound obtained in Example 1-1, 10.0 parts by mass of DMAA, 10.0 parts by mass of HBMA, 30.0 parts by mass of NVP, 30.0 parts by mass of SiGMA, 5.00 parts by mass of PVP K90, 1.00 part by mass of TEGDMA, and 30.0 parts by mass of HexOH were mixed. Subsequently, 0.50 part by mass of AIBN was added to the mixture, followed by mixing. Thus, a composition was produced.

The resultant composition was evaluated for its composition uniformity, and was given the score of "1".

Subsequently, the composition was dispensed into a contact lens mold, and the mold was placed in an oven. After the inside of the oven was purged with nitrogen, the temperature in the oven was raised to 100°C, and the inside of the oven was maintained at this temperature for 2 hours so that the composition was polymerized. Thus, a polymerization product was obtained.

The polymerization product was taken out from the mold, and was purified by being immersed in 40 g of 2-propanol for 4 hours and then immersed in 50 g of ion-exchanged water for 4 hours so that an unreacted substance and the like were removed. Further, the polymerization product was immersed in physiological saline described in ISO-18369-3 to provide a contact lens-shaped polymerization product.

When the contact lens-shaped polymerization product was evaluated for its polymerization product transparency, the polymerization product was given the score of "1". When the contact lens-shaped polymerization product was evaluated for its anti-lipid adhesion property, the polymerization product was given the score of "1". The results of Example 2-1 are shown in Table 2.

### [Examples 2-2 to 2-6]

Examples 2-2 to 2-6 were each performed in the same manner as in Example 2-1 except that the composition shown in Table 2 was followed. The evaluations of composition uniformity, polymerization product transparency, and an anti-lipid adhesion property were performed in the same manner as in Example 2-1.

The evaluation results of Examples 2-2 to 2-6 are shown in Table 2.

### [Comparative Example 2-1]

Comparative Example 2-1 was performed in the same manner as in Example 2-1 except that 20.0 parts by mass of the formula (1A) comparative compound prepared in Synthesis Example 1-5 was used instead of 20.0 parts by mass of the compound represented by the formula (1A) in accordance with the composition shown in Table 2. The evaluations of composition uniformity, polymerization product transparency, and an anti-lipid adhesion property were performed in the same manner as in Example 2-1. As a result, the score of "1" was given for the composition uniformity, the score of "1" was given for the polymerization product transparency, and the score of "5" was given for the anti-lipid adhesion property. The evaluation results of Comparative Example 2-1 are shown in Table 2.

### [Comparative Example 2-2]

A composition was produced in the same manner as in Example 2-1 except that 20.0 parts by mass of the formula (1A) comparative compound synthesized in Synthesis Example 1-6 was used instead of 20.0 parts by mass of the compound represented by the formula (1A) in accordance with the composition shown in Table 2.

The resultant composition was evaluated for its composition uniformity, and was given the score of "2". Thus, the composition was not subjected to the subsequent polymerization step and evaluation for its polymerization product transparency and anti-lipid adhesion property. The evaluation results of Comparative Example 2-2 are shown in Table 2.

### [Comparative Example 2-3]

Comparative Example 2-3 was performed in the same manner as in Example 2-1 except that 20.0 parts by mass of the formula (1A) comparative compound synthesized in Synthesis Example 1-7 was used instead of 20.0 parts by mass of the compound represented by the formula (1A) in accordance with the composition shown in Table 2. The evaluations of composition uniformity, polymerization product transparency, and an anti-lipid adhesion property were performed in the same manner as in Example 2-1. As a result, the score of "1" was given for the composition uniformity, the score of "4" was given for the polymerization product transparency, and the score of "4" was given for the anti-lipid adhesion property. The evaluation results of Comparative Example 2-3 are shown in Table 2.

**Table 2**

| Kind | | Raw materia 1 | Example 2-1 | Example 2-2 | Example 2-3 | Example 2-4 | Example 2-5 | Example 2-6 | Comparative Example 2-1 | Comparative Example 2-2 | Comparative Example 2-3 |
|---|---|---|---|---|---|---|---|---|---|---|---|
| | | | Mixing ratio | Mixing ratio | Mixing ratio | Mixing ratio | Mixing ratio | Mixing ratio | Mixing ratio | Mixing ratio | Mixing ratio |
| Formul a (1A) compou nd | | Example 1-1 | 20.0 | | | | | | | | |
| | | Example 1-2 | | 20.0 | | | | | | | |
| | | Example 1-3 | | | 20.0 | | | | | | |
| | | Example 1-4 | | | | 20.0 | | | | | |
| | | Example 1-5 | | | | | 20.0 | | | | |
| | | Example 1-6 | | | | | | 20.0 | | | |
| Formul a (1A) compar ative compou nd | Part by mass | Synthes is Example 1-5 | | | | | | | 20.0 | | |
| | | Synthes is Example 1-6 | | | | | | | | 20.0 | |
| | | Synthes is Example 1-7 | | | | | | | | | 20.0 |
| Hydrop hilic monome r | | DMAA | 10.0 | 10.0 | 10.0 | 10.0 | 10.0 | 10.0 | 10.0 | 10.0 | 10.0 |
| | | HEMA | | 10.0 | | | | | | | |
| | | HPMA | | | | 10.0 | | 10.0 | | | |
| | | HBMA | 10.0 | | 10.0 | | 10.0 | | 10.0 | 10.0 | 10.0 |
| | | NVP | 30.0 | 30.0 | 20.0 | 30.0 | 30.0 | 20.0 | 30.0 | 30.0 | 30.0 |
| | | MVA | | | | | | 10.0 | | | |
| | | MPC | | | 10.0 | | | | | | |
| Hydroxy group-containing siloxane monomer | | ETS | | | 30.0 | | | | | | |
| | | SiGMA | 30.0 | 30.0 | | 30.0 | 30.0 | 30.0 | 30.0 | 30.0 | 30.0 |
| Total of monome rs | | - | 100.0 | 100.0 | 100.0 | 100.0 | 100.0 | 100.0 | 100.0 | 100.0 | 100.0 |
| Hydrop hilic polyme r | | PVP K90 | 5.00 | 5.00 | 5.00 | 5.00 | 5.00 | 5.00 | 5.00 | 5.00 | 5.00 |
| Cross-linkin g agent | | TEGDMA | 1.00 | 1.00 | 1.00 | 1.00 | 1.00 | 1.00 | 1.00 | 1.00 | 1.00 |
| Solven t | | HexOH | 30.0 | 30.0 | 30.0 | 30.0 | 30.0 | 30.0 | 30.0 | 30.0 | 30.0 |
| Initia tor | | AIBN | 0.50 | 0.50 | 0.50 | 0.50 | 0.50 | 0.50 | 0.50 | 0.50 | 0.50 |
| Polymerization step | | Tempera ture | 100°C | 100°C | 100°C | 100°C | 100°C | 100°C | 100°C | - | 100°C |
| | | Time | 2 hours | 2 hours | 2 hours | 2 hours | 2 hours | 2 hours | 2 hours | - | 2 hours |

| Evaluation items | | | Evaluation results | | | | | | | | |
|---|---|---|---|---|---|---|---|---|---|---|---|
| Composition uniformity | | | 1 | 1 | 1 | 1 | 1 | 1 | 1 | 2 | 1 |
| Polymerization product transparency | | | 1 | 1 | 1 | 2 | 2 | 2 | 1 | - | 4 |
| Anti-lipid adhesion property | | | 1 | 1 | 1 | 1 | 2 | 3 | 5 | - | 4 |

The evaluation results in Table 2 are described below.

Examples 2-1 to 2-3 were each given the score of "1" for the composition uniformity and the score of "1" for the polymerization product transparency, and hence each exhibited significantly satisfactory compatibility with a hydrophilic monomer and a hydrophilic polymer.

Examples 2-4 to 2-6 were each given the score of "1" for the composition uniformity and the score of "2" for the polymerization product transparency, and hence each exhibited sufficiently satisfactory compatibility with a hydrophilic monomer and a hydrophilic polymer.

Examples 2-1 to 2-4 were each given the score of "1" for the anti-lipid adhesion property, and hence each exhibited a significantly high anti-lipid adhesion property.

Example 2-5 was given the score of "2" for the anti-lipid adhesion property, and hence exhibited a high anti-lipid adhesion property.

Example 2-6 was given the score of "3" for the anti-lipid adhesion property, and hence exhibited an anti-lipid adhesion property.

The above-mentioned results revealed that the compound represented by the formula (1A) used in each of Examples 2-1 to 2-6 simultaneously exhibited satisfactory compatibility with a hydrophilic monomer and a hydrophilic polymer and the imparting of an anti-lipid adhesion property to a polymerization product.

Comparative Example 2-1 was given the score of "1" for the composition uniformity and the score of "1" for the polymerization product transparency, and hence exhibited significantly satisfactory compatibility with a hydrophilic monomer and a hydrophilic polymer. Meanwhile, Comparative Example 2-1 was given the score of "5" for the anti-lipid adhesion property, and hence did not exhibit an anti-lipid adhesion property.

Comparative Example 2-2 was given the score of "2" for the composition uniformity, and hence did not exhibit satisfactory compatibility with a hydrophilic monomer and a hydrophilic polymer.

Comparative Example 2-3 was given the score of "1" for the composition uniformity but was given the score of "4" for the polymerization product transparency, and hence did not exhibit satisfactory compatibility with a hydrophilic monomer and a hydrophilic polymer. In addition, Comparative Example 2-3 was given the score of "4" for the anti-lipid adhesion property, and hence did not exhibit an anti-lipid adhesion property.

From the above-mentioned results, the formula (1A) comparative compound used in each of Comparative Examples 2-1 to 2-3 did not simultaneously exhibit satisfactory compatibility with a hydrophilic monomer and a hydrophilic polymer and an anti-lipid adhesion property.

It was recognized that the polydimethylsiloxane-containing monomer having a phosphorylcholine group and a hydroxy group represented by the formula (1A) exhibited satisfactory compatibility with a hydrophilic monomer and a hydrophilic polymer and imparted an anti-lipid adhesion property to a polymerization product obtained when its composition with the hydrophilic monomer and the hydrophilic polymer was polymerized.

### [Example 3-1]

2.00 g (40.0 parts by mass) of the formula (1A) compound obtained in Example 1-1, 1.00 g (20.0 parts by mass) of TRIS, 0.50 g (10.0 parts by mass) of MPC, and 1.10 g (22.0 parts by mass) of HBMA were mixed in a container, and were stirred at room temperature until the MPC was dissolved. Further, 0.4 g (8.0 parts by mass) of NVP, 0.05 g (1.0 part by mass) of TEGDMA, and 0.025 g (0.5 part by mass) of AIBN were added to the container, and the mixture was stirred at room temperature until the mixture became uniform. Thus, a composition was obtained.

The resultant composition was evaluated for its composition uniformity, and was given the score of "1". Subsequently, the composition was dispensed into a contact lens mold, and the mold was placed in an oven. After the inside of the oven was purged with nitrogen, the temperature in the oven was raised to 80°C, and the inside of the oven was maintained at 80°C for 12 hours so that the composition was polymerized. Thus, a polymerization product was obtained.

Each polymerization product was taken out from a cell to provide a polymerization product. The polymerization product was purified by being immersed in 100 mL of a 50 mass% 2-propanol aqueous solution for 4 hours and then immersed in 100 mL of ion-exchanged water for 4 hours so that an unreacted substance and the like were removed. The polymerization product after the purification was immersed in physiological saline described in ISO-18369-3 to be swollen (hydrated). Thus, a contact lens-shaped polymerization product was obtained. The contact lens-shaped polymerization product was subjected to the respective evaluations. The blending ratios of the respective components in the composition, polymerization conditions, and the respective evaluation results are shown in Table 3.

### [Examples 3-2 to 3-4]

Contact lens-shaped polymerization products were each obtained in the same manner as in Example 1 except that the blending ratios of the respective components in the composition and the polymerization conditions were set as shown in Table 3. The respective evaluations were performed in the same manner as in Example 3-1. The results are shown in Table 3.

In Example 3-2, the composition was polymerized by UV irradiation for 30 minutes at room temperature with a UV-LED irradiator (irradiation light wavelength: 405 nm) at an irradiance of 1.5 mW/cm² to provide a polymerization product.

### [Comparative Examples 3-1 to 3-3]

Contact lens-shaped polymerization products were each obtained in the same manner as in Example 1 except that the blending ratios of the respective components in the composition and the polymerization conditions were set as shown in Table 3. The respective evaluations were performed in the same manner as in Example 3-1. The results are shown in Table 3.

In Comparative Example 3-1, the resultant composition was evaluated for composition uniformity, and was given the score of "2". Thus, the subsequent polymerization step and respective evaluations were not performed.

In Comparative Example 3-3, the resultant polymerization product was evaluated for polymerization product transparency, and was given the score of "2". Thus, the subsequent respective evaluations were not performed.
TRIS: 3-[tris(trimethylsiloxy)silyl]propyl methacrylate
I-189: bis(2,4,6-trimethylbenzoyl)-phenylphosphineoxide
EtOH: ethanol
NPA: 1-propanol (normal propyl alcohol)

**Table 3**

| Kind | | | Example 3-1 | Example 3-2 | Example 3-3 | Example 3-4 | Comparative Example 3-1 | Comparative Example 3-2 | Comparative Example 3-3 |
|---|---|---|---|---|---|---|---|---|---|
| Formula (1A) compound | Part (s) by mass | Example 1-1 | 40.0 | 40.0 | 0.0 | 0.0 | 0.0 | 0.0 | 0.0 |
| | | Example 1-5 | 0.0 | 0.0 | 35.0 | 15.0 | 0.0 | 0.0 | 0.0 |
| Formula (1A) comparative compound | | Synthesis Example 1-7 | 0.0 | 0.0 | 0.0 | 0.0 | 0.0 | 0.0 | 15.0 |
| Siloxane monomer | | MCR-M11 | 0.0 | 0.0 | 0.0 | 25.0 | 40.0 | 30.0 | 25.0 |
| | | TRIS | 20.0 | 0.0 | 0.0 | 0.0 | 20.0 | 0.0 | 0.0 |
| | | ETS | 0.0 | 20.0 | 20.0 | 0.0 | 0.0 | 0.0 | 0.0 |
| | | SiGMA | 0.0 | 0.0 | 0.0 | 20.0 | 0.0 | 20.0 | 20.0 |
| Hydrophilic monomer | | MPC | 10.0 | 11.0 | 11.0 | 15.0 | 10.0 | 0.0 | 15.0 |
| | | HEMA | 0.0 | 15.0 | 0.0 | 0.0 | 0.0 | 20.0 | 0.0 |
| | | HBMA | 22.0 | 0.0 | 23.0 | 20.0 | 22.0 | 0.0 | 20.0 |
| | | NVP | 8.0 | 14.0 | 11.0 | 5.0 | 8.0 | 30.0 | 5.0 |
| Total of monomers | | - | 100.0 | 100.0 | 100.0 | 100.0 | 100.0 | 100.0 | 100.0 |
| Cross-linking agent | | TEGDMA | 1.0 | 1.0 | 1.0 | 1.0 | 0.5 | 0.5 | 0.5 |
| Initiator | | AIBN | 0.5 | 0.0 | 0.0 | 0.5 | 0.5 | 0.5 | 0.5 |
| | | I-189 | 0.0 | 0.5 | 0.5 | 0.0 | 0.0 | 0.0 | 0.0 |
| Solvent | | EtOH | 0.0 | 0.0 | 6.0 | 0.0 | 0.0 | 0.0 | 0.0 |
| | | NPA | 0.0 | 10.0 | 0.0 | 10.0 | 0.0 | 20.0 | 20.0 |
| Polymerization conditions | Method | | Heat | Light | Heat | Heat | - | Heat | Heat |
| | Temperature | | 80 | r.t. | 80 | 80 | - | 80 | 80 |
| | Irradiance (mW/cm²) | | - | 1.5 | - | - | - | - | - |
| | Time (hrs) | | 12 | 0.5 | 12 | 12 | - | 12 | 12 |
| Evaluation scores | Transparency of composition | | 1 | 1 | 1 | 1 | 2 | 1 | 1 |
| | Transparency of contact lens | | 1 | 1 | 1 | 1 | - | 1 | 2 |
| | WBUT (s) | 1 (>10) | 1 (>10) | 1 (>10) | 1 (>10) | - | 2 (5) | - | |
| | Antifouling property | 1 | 1 | 1 | 2 | - | 5 | - | |

The evaluation results in Table 3 are described below.

Examples 3-1 to 3-4 were each given the score of "1" for composition uniformity and the score of "1" for polymerization product transparency, and hence each exhibited significantly satisfactory compatibility with a hydrophilic monomer.

Examples 3-1 to 3-4 were each given the score of "1" in the evaluation of wettability (evaluation of hydrophilicity), and hence each exhibited significantly high hydrophilicity.

Examples 3-1 to 3-3 were each given the score of "1" in the evaluation of an antifouling property (evaluation of coatability), and hence each exhibited significantly high coatability. Example 3-4 was given the score of "2" in the evaluation of an antifouling property (evaluation of coatability), and hence exhibited high coatability.

From the above-mentioned results, it was recognized that the compound represented by the formula (1A) used in each of Examples 3-1 to 3-4 simultaneously exhibited satisfactory compatibility with a hydrophilic monomer and the imparting of hydrophilicity and coatability to a polymerization product.

Comparative Example 3-2 was given the score of "5" in the evaluation of an antifouling property (evaluation of coatability), and hence did not exhibit coatability.

It was recognized that the polydimethylsiloxane-containing monomer having a phosphorylcholine group and a hydroxy group represented by the formula (1A) exhibited satisfactory compatibility with a hydrophilic monomer and a hydrophilic polymer and imparted an anti-lipid adhesion property, hydrophilicity, and coatability to a polymerization product when its composition with the hydrophilic monomer and the hydrophilic polymer was polymerized to provide the polymerization product.

### Industrial Applicability

The present disclosure can provide a polydimethylsiloxane-containing monomer having a phosphorylcholine group and a hydroxy group, the monomer having the following characteristics.
(1) The compatibility with a hydrophilic monomer and a hydrophilic polymer is satisfactory.
(2) A polymerization product obtained by polymerizing a composition containing the monomer of the present disclosure, a hydrophilic monomer, and a hydrophilic polymer has transparency and an anti-lipid adhesion property.
(3) A polymerization product obtained by polymerizing a composition containing the monomer of the present disclosure and a hydrophilic monomer has transparency and excellent surface hydrophilicity and coatability without surface treatment.

## Claims

1. A compound represented by the formula (1A): 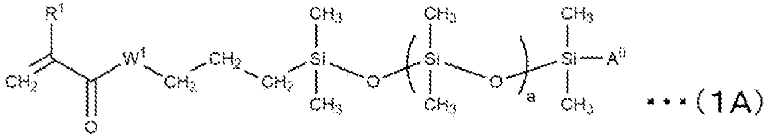 where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 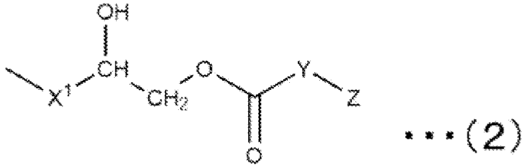 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 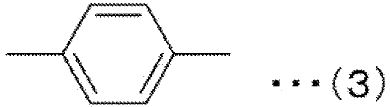 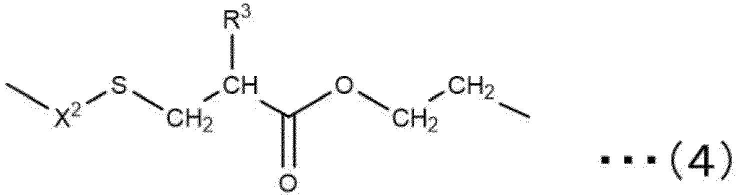 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.

2. A method of producing a compound represented by the formula (1A),
the method comprising a step of synthesizing the formula (1A) by:
a hydrosilylation reaction between a compound represented by the formula (6A) and a vinyl compound having an epoxy group, and further a reaction between an epoxy group of a compound after the reaction and a carboxylic acid of a carboxylic acid compound having a phosphorylcholine group; or
a reaction between an epoxy group of a vinyl compound having an epoxy group and a carboxylic acid of a carboxylic acid compound having a phosphorylcholine group, and further a hydrosilylation reaction between a vinyl compound having a phosphorylcholine group and a hydroxy group after the reaction and the compound represented by the formula (6A): 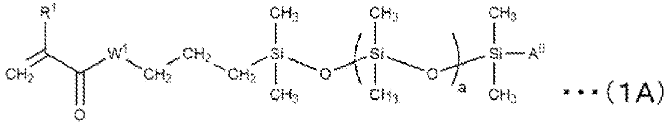
where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 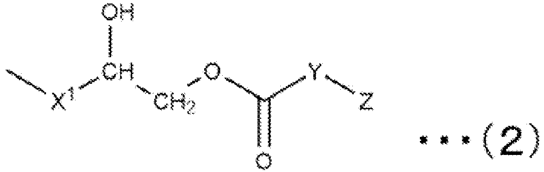 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 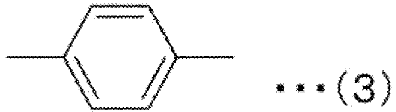 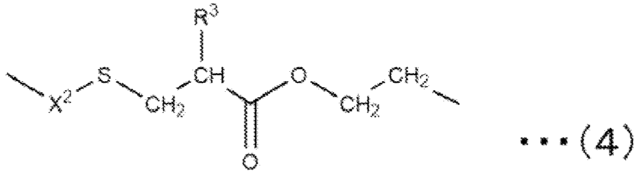 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms; 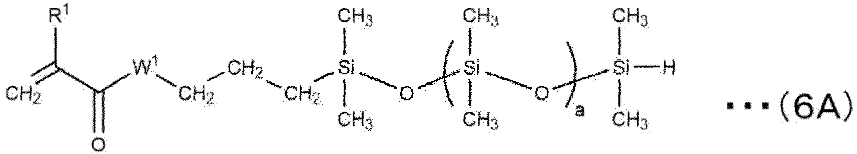 where R¹ represents H or CH₃, W¹ represents O or NR⁴, where R⁴ represents H or an alkyl group having 1 to 4 carbon atoms, and "a" represents an integer of from 1 to 100.

3. A method of producing a polydimethylsiloxane-containing monomer having a hydrosilane at a terminal thereof represented by the formula (6A),
the method comprising a step of synthesizing the polydimethylsiloxane-containing monomer having a hydrosilane at a terminal thereof represented by the formula (6A) by:
converting a chlorosilane compound represented by the formula (7) into a silanol compound represented by the formula (7A) with a metal hydroxide; and
then allowing the silanol compound to react with a cyclic siloxane and a chlorosilane compound in the presence of an organic base: 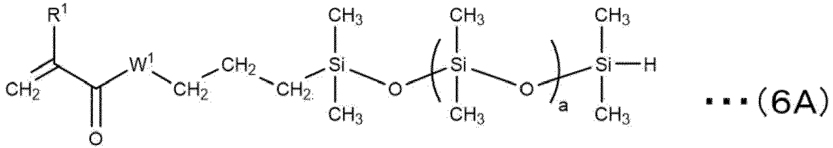
where R¹ represents H or CH₃, W¹ represents O or NR⁴, where R⁴ represents H or an alkyl group having 1 to 4 carbon atoms, and "a" represents an integer of from 1 to 100; 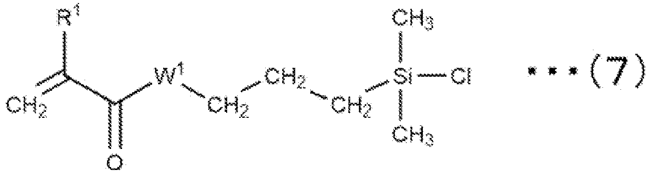 where R¹ represents H or CH₃, W¹ represents O or NR⁴, where R⁴ represents H or an alkyl group having 1 to 4 carbon atoms; 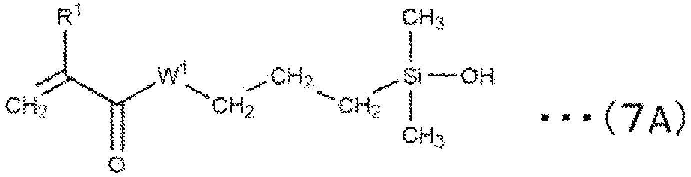 where R¹ represents H or CH₃, W¹ represents O or NR⁴, where R⁴ represents H or an alkyl group having 1 to 4 carbon atoms.

4. A composition, comprising:
a compound represented by the following formula (1A); and
one or more kinds of hydrophilic monomers: 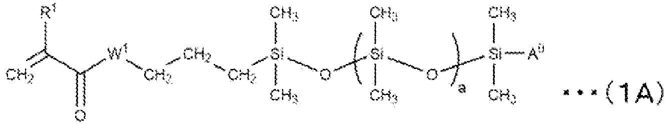
where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 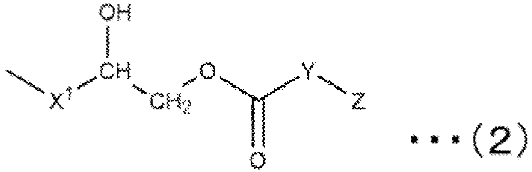 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 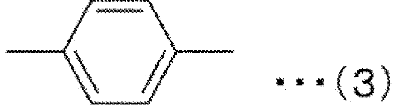 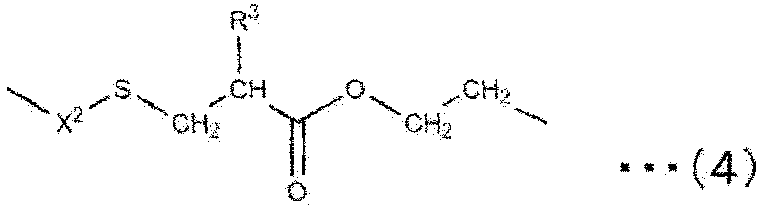 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.

5. A composition, comprising:
a compound represented by the following formula (1A);
one or more kinds of hydrophilic monomers; and
one or more kinds of hydrophilic polymers: 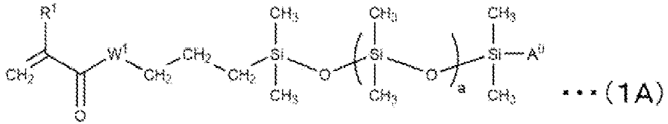
where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 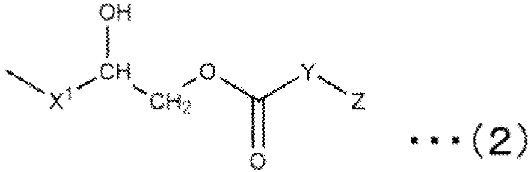 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 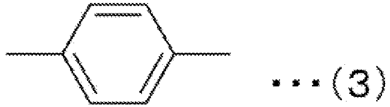 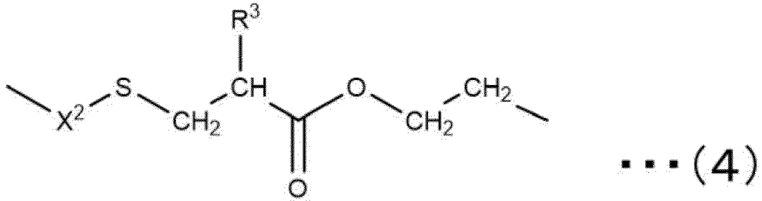 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.

6. A composition, comprising:
a compound represented by the following formula (1A);
one or more kinds of hydrophilic monomers; and
one or more kinds of siloxane monomers: 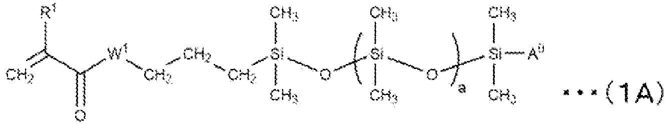
where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 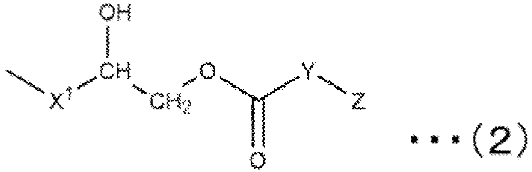 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 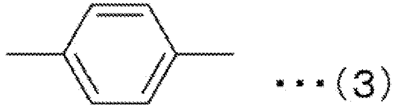 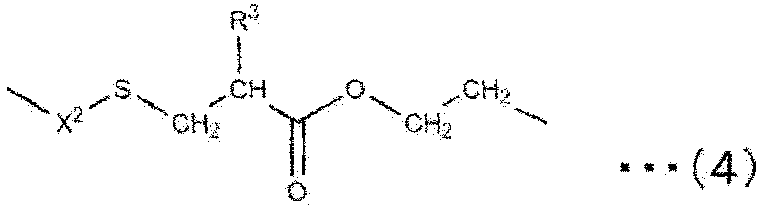 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.

7. The composition according to any one of claims 4 to 6, wherein the hydrophilic monomer is 2-(methacryloyloxyethyl)-2-(trimethylammonioethyl) phosphate.

8. A composition, comprising:
a compound represented by the following formula (1A);
one or more kinds of hydrophilic monomers;
one or more kinds of hydroxy group-containing siloxane monomers; and
one or more kinds of hydrophilic polymers. 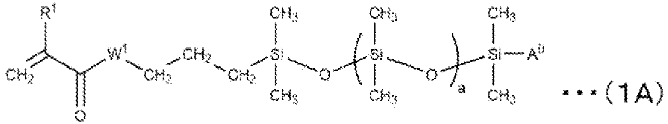
where R¹ represents H or CH₃, W¹ represents O or NR⁴, where R⁴ represents H or an alkyl group having 1 to 4 carbon atoms, "a" represents an integer of from 1 to 100, and A⁰ is represented by the formula (2); 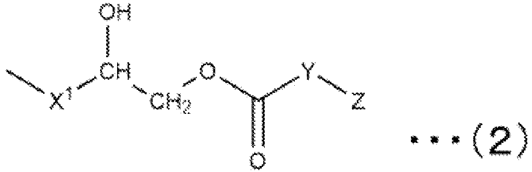 where X¹ represents a divalent alkylene group having 3 to 8 carbon atoms or a divalent group of -R⁶-O-R⁷-, where R⁶ represents a divalent alkylene group having 3 to 6 carbon atoms and R⁷ represents a divalent alkylene group having 1 to 6 carbon atoms, Y represents a divalent alkylene group having 1 to 8 carbon atoms or a divalent group of -R⁸-O-R⁹-, where R⁸ and R⁹ each independently represent a divalent alkylene group having 1 to 6 carbon atoms, or Y is represented by the formula (3) or the formula (4), and Z represents a phosphorylcholine group; 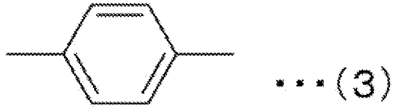 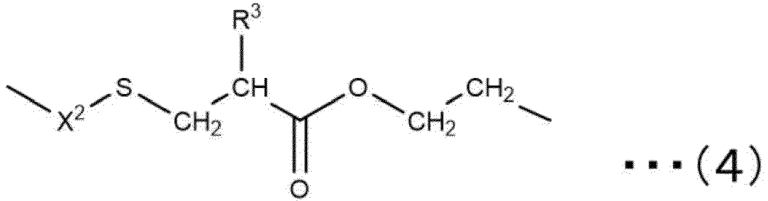 where R³ represents H or CH₃, X² represents a divalent alkylene group having 1 to 10 carbon atoms or a divalent group of -R¹⁰-OR¹¹-, where R¹⁰ and R¹¹ each independently represent a divalent alkylene group having 1 to 6 carbon atoms.

9. The composition according to any one of claim 5 or 8, wherein the hydrophilic polymer is one or more selected from the group consisting of: polyamide; polylactam; polyimide; polylactone; and polydextran.

10. The composition according to any one of claim 5 or 8, wherein the hydrophilic polymer is polyvinylpyrrolidone.

11. A polymerization product obtained by polymerizing the composition of any one of claims 4 to 6 and 8.

12. An ophthalmic device, comprising the polymerization product of claim 11.
